(19) **Europäisches Patentamt / European Patent Office / Office européen des brevets**

(11) **EP 1 977 931 A1**

(12) **EUROPEAN PATENT APPLICATION**
published in accordance with Art. 158(3) EPC

(43) Date of publication:
**08.10.2008 Bulletin 2008/41**

(21) Application number: **07707340.1**

(22) Date of filing: **24.01.2007**

(51) Int Cl.:
**B60R 11/02** (2006.01)    **B60R 16/02** (2006.01)
**G09G 5/00** (2006.01)    **G09G 5/36** (2006.01)
**H04N 5/66** (2006.01)

(86) International application number:
**PCT/JP2007/051093**

(87) International publication number:
**WO 2007/086431 (02.08.2007 Gazette 2007/31)**

(84) Designated Contracting States:
**DE FR GB**

(30) Priority: **25.01.2006 JP 2006015915**

(71) Applicant: **MATSUSHITA ELECTRIC INDUSTRIAL CO., LTD.**
**Osaka 571-8501 (JP)**

(72) Inventors:
• **MORIMOTO, Akihiro c/o Matsuhita E. Ind. Co., Ltd.**
**Osaka 540-6207 (JP)**

• **ISU, Naoki c/o Faculty of Engineering**
**Mie University**
**Mie 514-8507 (JP)**

(74) Representative: **Balsters, Robert et al**
**Novagraaf SA**
**25, Avenue du Pailly**
**1220 Les Avanchets - Geneva (CH)**

(54) **VIDEO DISPLAY**

(57)    An image display device capable of reducing the burden on a passenger and reducing the occurrence of motion sickness, by giving the passenger, through a visual sense, perception (visually induced self-motion perception) of his/her own body moving while viewing an image of a TV and the like in a vehicle, and thus by matching visual information to vestibular information obtained from the motion of the vehicle, particularly to a sense of rotation and somatosensory information which are detected by his/her semicircular canals, is provided. The image display device includes: a behavior detection section (101) for detecting a behavior of a vehicle; a background image generation section (102) for generating a background image based on the behavior detected by the behavior detection section (101); an image generation section (103) for generating an image; an image transformation section (104) for, based on the behavior detected by the behavior detection section (101), transforming the image generated by the image generation section (103); a composition section (105) for making a composite image of the background image generated by the background image generation section (102) and the image transformed by the image transformation section (104) ; and a display section (106) for displaying the composite image made by the composition section (105).

F I G. 1

**Description**

TECHNICAL FIELD

The present invention relates to an image display device, and particularly to an image display device for providing a passenger of a vehicle with an image.

BACKGROUND ART

In recent years, a growing number of vehicles each have mounted thereon a display for displaying a wide variety of information. Particularly, a growing number of vehicles each have mounted thereon a display used for a navigation device for displaying a map, the center of which is the vehicle's position. Further, also a growing number of vehicles each have mounted thereon a display for displaying images of a TV (Television), a VTR (Video Tape Recorder), a DVD (Digital Versatile Disk), a movie, a game, and the like for its passenger seat and its back seat.

At the same time, inside a vehicle such as an automobile, there exist: a vibration caused by the engine and other drive mechanisms of the vehicle; a vibration received by the chassis of the vehicle from the outside of the vehicle and caused by a road terrain, an undulation, a road surface condition, a curb, and the like while the steered vehicle is traveling; a vibration caused by a shake, an impact, and like; and a vibration caused by acceleration and braking of the vehicle.

A sensory discrepancy theory (a sensory conflict theory, a neural mismatch theory) is known in which, when a person rides in such a vehicle and the like, the actual pattern of sensory information obtained when he/she is placed in a new motion environment is different from the pattern of sensory information stored in his/her central nervous system, and therefore the central nervous system is confused by not being able to recognize its own position or motion (see Non-patent Document 1, for example). In this case, the central nervous system recognizes the new pattern of sensory information, and it is considered that motion sickness (carsickness) occurs during an adaptation process of the recognition. For example, when a person reads a book in a vehicle, the line of his/her vision is fixed. Consequently, visual information does not match vestibular information obtained from the motion of the vehicle, and particularly does not match a sense of rotation and somatosensory information which are detected by his/her semicircular canals, and as a result, motion sickness occurs. To avoid a sensory conflict between the visual information and the vestibular information, it is considered good to close his/her eyes or look off far in the distance when in the vehicle. Further, it is considered that the reason that a driver is less likely to suffer from motion sickness than a passenger is is that the driver is tense from driving and also that the driver, in anticipation of the motion of the vehicle, actively positions his/her head so that the head is least changed by the acceleration.

As a countermeasure for such motion sickness, a method is proposed for allowing a passenger other than a driver to recognize the current motion of the vehicle and to anticipate the next motion thereof, by indicating the left/right turns, the acceleration/deceleration, and the stop of the vehicle (see Patent Document 1, for example).

Further, to reduce motion sickness of a backseat passenger, a method is also proposed for informing the backseat passenger through an auditory sense or a visual sense that the brake will be applied on the vehicle or that the vehicle will turn left/right, by providing audio guidance such as "the car will decelerate" or "the car will turn right" and by displaying a rightward arrow when the vehicle turns right, in response to operation information from the steering wheel, the brake, and the turn signal (see Patent Document 2, for example). Non-patent Document 1: Toru Matsunaga, Noriaki Takeda: Motion Sickness and Space Sickness, Practica Oto-Rhino-Laryngologica, Vol. 81, No. 8, pp. 1095 - 1120, 1998 Patent Document 1: Japanese Laid-Open Patent Publication No. 2002-154350 (FIG. 1) Patent Document 2: Japanese Laid-Open Patent Publication No. 2003-154900

DISCLOSURE OF THE INVENTION

PROBLEMS TO BE SOLVED BY THE INVENTION

However, based on the motion sickness countermeasures of display devices disclosed in Patent Document 1 and Patent Document 2, the passenger is merely informed, based on operation information from the steering wheel, the brake, and the turn signal, that the vehicle will accelerate/decelerate or turn left/right, and therefore the passenger requires two steps: one for recognizing the motion of the vehicle from the given information and the other for bracing himself/herself for the recognized motion. Consequently, even when the passenger is informed that the vehicle will accelerate/decelerate or turn left/right, the passenger does not necessarily brace himself/herself as a result, and thus it is impossible to sufficiently prevent motion sickness from occurring.

The present invention is directed to solving the above problems. That is, an object of the present invention is to provide an image display device capable of reducing the burden on a passenger and reducing the occurrence of motion sickness, by giving the passenger, through a visual sense, perception (visually induced self-motion perception) of his/her own body moving while viewing an image of a TV and the like in a vehicle, and thus by matching visual information to vestibular information obtained from the motion of the vehicle, particularly to a sense of rotation and somatosensory information which are detected by his/her semicircular canals.

SOLUTION TO THE PROBLEMS

A first aspect of the present invention is directed to an image display device. The image display device of the present invention includes: a behavior detection section for detecting a behavior of a vehicle; a background image generation section for generating a background image based on the behavior detected by the behavior detection section; an image transformation section for transforming an image based on the behavior of the vehicle which is detected by the behavior detection section; a composition section for making a composite image of the background image generated by the background image generation section and the image transformed by the image transformation section; and a display section for displaying the composite image made by the composition section.

Based on the above-described structure, it is possible to provide the image display device capable of reducing the burden on a passenger and reducing the occurrence of motion sickness, by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

Further, it is preferable that the behavior detection section detects the behavior of the vehicle, using at least one of signals of a velocity sensor, an acceleration sensor, and an angular velocity sensor.

Based on the above-described structure, it is possible to certainly detect the behavior such as acceleration/deceleration, an acceleration, and an angular velocity, each applied to the vehicle.

Further, it is preferable that the behavior detection section detects the behavior of the vehicle based on a state of an operation performed on the vehicle by a driver of the vehicle.

Based on the above-described structure, the behavior is detected based on the state of the operation such as steering and braking, each performed on the vehicle by the driver, whereby it is possible to certainly detect the behavior such as left/right turns and acceleration/deceleration, each applied to the vehicle.

Further, it is preferable that the behavior detection section detects the behavior of the vehicle based on an output from a capture section for capturing an external environment of the vehicle.

Based on the above-described structure, it is possible to easily recognize road information related to the forward traveling direction of the vehicle, whereby it is possible to anticipate the behavior of the vehicle.

Further, it is preferable that the behavior detection section detects the behavior of the vehicle based on an output from a navigation section for providing route guidance for the vehicle.

Based on the above-described structure, it is possible to easily recognize road information related to the forward traveling direction of the vehicle, whereby it is possible to anticipate the behavior of the vehicle.

Further, it is preferable that the behavior detection section detects one or more of a leftward/rightward acceleration, an upward/downward acceleration, a forward/backward acceleration, and an angular velocity of the vehicle.

Based on the above-described structure, it is possible to detect the combined behavior of the vehicle.

Further, it is preferable that the background image generation section changes a display position of the background image in accordance with the behavior of the vehicle which is detected by the behavior detection section.

Based on the above-described structure, it is possible to reduce the burden on the passenger by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

Further, it is preferable that in accordance with the behavior of the vehicle which is detected by the behavior detection section, the background image generation section generates the background image moved to the right when the behavior indicates a left turn and also generates the background image moved to the left when the behavior indicates a right turn.

Based on the above-described structure, it is possible to reduce the burden on the passenger by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

Further, it is preferable that the background image generation section generates a vertical stripe pattern as the background image.

Based on the above-described structure, the vertical stripe pattern as the background image is moved to the left or to the right, whereby it is possible for the passenger to easily recognize the leftward/rightward behavior of the vehicle as the visual information.

Further, it is preferable that in accordance with the behavior of the vehicle which is detected by the behavior detection section, the background image generation section generates the background image rotated to the left when the behavior indicates a left turn and also generates the background image rotated to the right when behavior indicates a right turn.

Based on the above-described structure, it is possible to reduce the burden on the passenger by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

Further, it is preferable that the image transformation section trapezoidal-transforms the image in accordance with the behavior of the vehicle which is detected by the behavior detection section.

Based on the above-described structure, it is possible to reduce the burden on the passenger by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

Further, it is preferable that in accordance with the behavior of the vehicle which is detected by the behavior detection section, the image transformation section trapezoidal-transforms the image by performing any of an enlargement and a reduction of at least one of a left end, a right end, a top end, and a bottom end of the image.

Based on the above-described structure, it is possible to reduce the burden on the passenger by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

Further, it is preferable that the image transformation section enlarges or reduces the image.

Based on the above-described structure, it is possible to reduce the burden on the passenger by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

Further, it is preferable that the composition section makes the composite image such that the background image generated by the background image generation section is placed in a background and the image transformed by the image transformation section is placed in a foreground.

Based on the above-described structure, it is possible to reduce the burden on the passenger by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

Further, it is preferable that in accordance with the behavior of the vehicle which is detected by the behavior detection section, the composition section changes display positions of the background image generated by the background image generation section and of the image transformed by the image transformation section.

Based on the above-described structure, it is possible to reduce the burden on the passenger by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

Further, it is preferable that the image display device of the present invention further includes a background image setting section for setting the back ground image generation section for generating the background image.

Based on the above-described structure, the background image setting section can set the level of visually induced self-motion perception for the passenger by setting the display position of the background image to be generated.

Further, it is preferable that the background image setting section selects a type of the background image.

Based on the above-described structure, the background image setting section can set the type of the background image to be generated by the background image generation section for the passenger.

Further, it is preferable that based on the behavior of the vehicle which is detected by the behavior detection section, the background image setting section sets a degree of changing a display position of the background image.

Based on the above-described structure, the background image setting section can set the level of visually induced self-motion perception for the passenger by changing the display position of the background image.

Further, it is preferable that based on the behavior of the vehicle which is detected by the behavior detection section, the background image setting section changes and sets, depending on a display position provided on the display section, the degree of changing the display position of the background image.

Based on the above-described structure, the background image setting section can set the level of visually induced self-motion perception for the passenger by changing the display position of the background image.

Further, it is preferable that the image display device of the present invention further includes an image transformation setting section for setting the image transformation section for transforming the image.

Based on the above-described structure, the image transformation setting section can set the level of visually induced self-motion perception for the passenger by setting the shape of the image to be transformed.

Further, it is preferable that the image transformation setting section sets the image transformation section to perform any one of a trapezoidal transformation, a reduction, and no transformation on the image to be transformed.

Based on the above-described structure, the image transformation setting section can set the level of visually induced self-motion perception for the passenger by setting the shape of the image to be transformed.

Further, it is preferable that when the image transformation section is set to perform the trapezoidal transformation on the image to be transformed, the image transformation setting section sets a shape and a reduction ratio of the trapezoid.

Based on the above-described structure, the image transformation setting section can set the level of visually induced self-motion perception for the passenger by setting the shape and the reduction ratio of the trapezoid for the transformation to be performed by the image transformation section.

Further, it is preferable that based on the behavior of the vehicle which is detected by the behavior detection section, the image transformation setting section sets a degree of transforming the image.

Based on the above-described structure, the image transformation setting section can set the level of visually induced self-motion perception for the passenger by setting the degree of the transformation to be performed by the image transformation section.

A second aspect of the present invention is directed to an image display device. The image display device of the present invention includes: a behavior detection section for detecting a behavior of a vehicle; a background image generation section for generating a background image which moves based on the behavior detected by the behavior detection section; an image transformation section for reducing an image; a composition section for making a composite image of the background image generated by the background image generation section and the image reduced by the image transformation section; and a display section for displaying the composite image made by the composition section.

Based on the above-described structure, it is possible to provide the image display device capable of reducing the burden on a passenger and reducing the occurrence of motion sickness, by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

A third aspect of the present invention is directed to an image display device. The image display device of the present invention includes: a behavior detection section for detecting a behavior of a vehicle; an image transformation section for transforming an image based on the behavior detected by the behavior detection section; and a display section for displaying the image transformed by the image transformation section.

Based on the above-described structure, it is possible to provide the image display device capable of reducing the burden on a passenger and reducing the occurrence of motion sickness, by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

Further, it is preferable that a vehicle of the present invention includes the above-described image display device.

Based on the above-described structure, it is possible to reduce the burden on the passenger by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

EFFECT OF THE INVENTION

As described above, the present invention can reduce the burden on a passenger by giving the passenger, through a visual sense, perception (visually induced self-motion perception) of his/her own body moving while viewing an image of a TV and the like in a vehicle, and thus by matching visual information to vestibular information obtained from the motion of the vehicle, particularly to a sense of rotation and somatosensory information which are detected by his/her semicircular canals. Further, consequently, it is possible to reduce the occurrence of motion sickness.

BRIEF DESCRIPTION OF THE DRAWINGS

[FIG. 1] FIG. 1 is a block diagram showing an overall structure of an image display device according to a first embodiment of the present invention.

[FIG. 2] FIG. 2 is a diagram showing an example of display performed by a display section according to the first embodiment of the present invention.

[FIG. 3] FIG. 3 is a diagram illustrating an angular velocity and a centrifugal acceleration both generated while a vehicle is traveling along a curve in the first embodiment of the present invention.

[FIG. 4] FIG. 4 is a diagram showing a relationship between an angular velocity $\omega$ outputted from a behavior detection section according to the first embodiment of the present invention and a moving velocity u of a background image outputted from a background image generation section according to the first embodiment of the present invention.

[FIG. 5] FIG. 5 is a diagram showing another example of the relationship between the angular velocity $\omega$ outputted from the behavior detection section according to the first embodiment of the present invention and the moving velocity u of the background image outputted from the background image generation section according to the first embodiment of the present invention.

[FIG. 6] FIG. 6 is a diagram showing a relationship between the angular velocity $\omega$ outputted from the behavior detection section according to the first embodiment of the present invention and the moving velocity u of the background image outputted from the background image generation section according to the first embodiment of the present invention.

[FIG. 7] FIG. 7 is a diagram showing another example of the display performed by the display section according to the first embodiment of the present invention.

[FIG. 8] FIG. 8 is a flow chart showing the flow of the operation of the image display device according to the first embodiment of the present invention.

[FIG. 9] FIG. 9 is: (a) a diagram showing an experimental result of a yaw angular velocity generated while a vehicle is traveling in the first embodiment of the present invention; and (b) a diagram showing an experimental result of the yaw angular velocity generated while the vehicle is traveling typical intersections in the first embodiment of the present invention.

[FIG. 10] FIG. 10 is a diagram showing an experimental result used for describing the effect of the image display device according to the first embodiment of the present invention.

[FIG. 11] FIG. 11 is a diagram showing an experimental result used for describing the effect of the image display device according to the first embodiment of the present invention.

[FIG. 12] FIG. 12 is a diagram showing an example of display performed by the display section according to the first embodiment of the present invention.

[FIG. 13] FIG. 13 is a diagram showing an experimental result used for describing the effect of the image display device according to the first embodiment of the present invention.

[FIG. 14] FIG. 14 is a diagram showing examples of display performed by a display section according to a second embodiment of the present invention.

[FIG. 15] FIG. 15 is a diagram showing a relationship between: an angular velocity $\omega$ outputted from a behavior detection section according to the second embodiment of the present invention; and a ratio k between the left end and the right end of an image trapezoidal-transformed by an image transformation section according to the second embodiment of the present invention.

[FIG. 16] FIG. 16 is a diagram showing another example of the relationship between: the angular velocity $\omega$ outputted from the behavior detection section according to the second embodiment of the present invention; and the ratio k between the left end and the right end of the image trapezoidal-transformed by the image transformation section according to the second embodiment of the present invention.

[FIG. 17] FIG. 17 is a diagram showing a relationship between: the angular velocity $\omega$ outputted from the behavior detection section according to the second embodiment of the present invention; and the ratio k between the left end and the right end of the image trapezoidal-transformed by the image transformation section according to the second embodiment of the present invention.

[FIG. 18] FIG. 18 is: (a) a diagram showing a front elevation view of the display section; and (b) a diagram showing a bird' s-eye view of the display section, both of which illustrate a method of the image transformation section

**EP 1 977 931 A1**

trapezoidal-transforming an image in the second embodiment of the present invention.

[FIG. 19] FIG. 19 is a diagram showing a relationship between: the angular velocity ω outputted from the behavior detection section according to the second embodiment of the present invention; and a ratio m of the top/bottom ends of the image as compared before and after the trapezoidal transformation performed by the image transformation section according to the second embodiment of the present invention.

[FIG. 20] FIG. 20 is a diagram showing another example of the relationship between: the angular velocity ω outputted from the behavior detection section according to the second embodiment of the present invention; and the ratio m of the top/bottom ends of the image as compared before and after the trapezoidal transformation performed by the image transformation section according to the second embodiment of the present invention.

[FIG. 21] FIG. 21 is a diagram showing a relationship between: the angular velocity ω outputted from the behavior detection section according to the second embodiment of the present invention; and the ratio m of the top/bottom ends of the image as compared before and after the trapezoidal transformation performed by the image transformation section according to the second embodiment of the present invention.

[FIG. 22] FIG. 22 is a flow chart showing the flow of the operation of the image display device according to the second embodiment of the present invention.

[FIG. 23] FIG. 23 is a diagram showing an experimental result used for describing the effect of the image display device according to the second embodiment of the present invention.

[FIG. 24] FIG. 24 is a diagram showing an experimental result used for describing the effect of the image display device according to the second embodiment of the present invention.

[FIG. 25] FIG. 25 is a diagram showing an example of display performed by a display section according to a third embodiment of the present invention.

[FIG. 26] FIG. 26 is a diagram showing another example of the display performed by the display section according to the third embodiment of the present invention.

[FIG. 27] FIG. 27 is a flow chart showing the flow of the operation of the image display device according to the third embodiment of the present invention.

[FIG. 28] FIG. 28 is a diagram showing an experimental result used for describing the effect of the image display device according to the third embodiment of the present invention.

[FIG. 29] FIG. 29 is a diagram showing an experimental result used for describing the effect of the image display device according to the third embodiment of the present invention.

DESCRIPTION OF THE REFERENCE CHARACTERS

101 behavior detection section
102 background image generation section
103 image generation section
104 image transformation section
105 composition section
106 display section
107 navigation section
108 capture section
109 background image setting section
110 image transformation setting section
201, 1401, 1403, 1405, 1407, 1802, 1803, 2501 image
202, 702, 1202, 1402, 1404, 1406, 1408, 2502, 2602 background image
301 vehicle
401, 402, 403, 501, 502, 503, 601, 602, 603 relationship between angular velocity and moving velocity of background image
901, 902 angular velocity
1501, 1502, 1503, 1601, 1602, 1603, 1701, 1702, 1703 relationship between angular velocity and ratio between left end and right end
1801 display section
1804 central axis
1805, 1806 virtual screen
1807 virtual camera
1901, 1902, 1903, 2001, 2002, 2003, 2101, 2102, 2103 relationship between: angular velocity; and ratio of top/bottom ends as compared before and after trapezoidal transformation

BEST MODE FOR CARRYING OUT THE INVENTION

With reference to the drawings, an image display device according to each embodiment of the present invention will be described in detail below.

(First Embodiment)

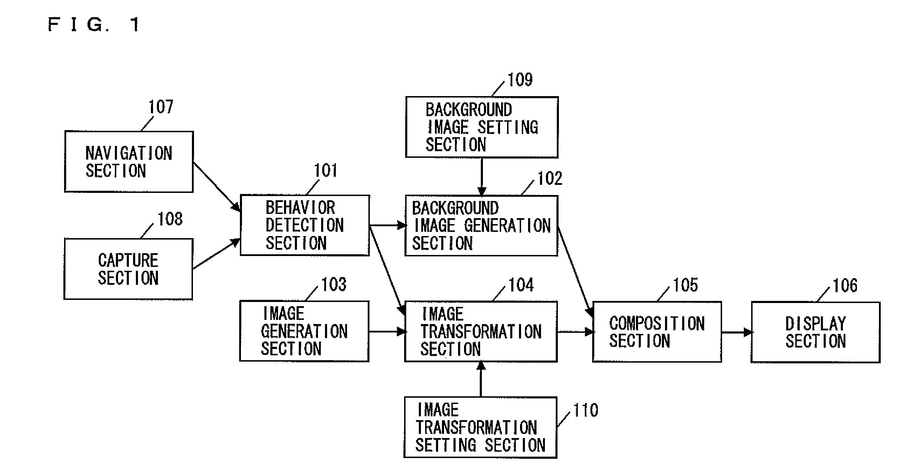

FIG. 1 is a block diagram showing an overall structure of an image display device according to a first embodiment of the present invention. Referring to FIG. 1, the image display device includes: a behavior detection section 101 for detecting the behavior of a vehicle; a background image generation section 102 for generating a background image based on the behavior detected by the behavior detection section 101; an image generation section 103 for generating an image; an image transformation section 104 for, based on the behavior detected by the behavior detection section 101, transforming the image generated by the image generation section 103; a composition section 105 for making a composite image of the background image generated by the background image generation section 102 and the image transformed by the image transformation section 104; a display section 106 for displaying the composite image made by the composition section 105; a navigation section 107 for providing route guidance for the vehicle; a capture section 108 for capturing the periphery of the vehicle; a background image setting section 109 for setting the background image generation section 102; and an image transformation setting section 110 for setting the image transformation section 104.

The behavior detection section 101 detects at least one of the upward/downward acceleration, the leftward/ rightward acceleration, the forward/backward acceleration, and the angular velocity of the vehicle, by using any one of acceleration/deceleration sensed by a velocity sensor, acceleration/deceleration sensed by an acceleration sensor, and an angular velocity (pitching, rolling, and yawing) sensed by an angular velocity sensor.

Further, the behavior detection section 101 may detect the behavior of the vehicle also based on the state of an operation performed on the vehicle by a driver. For example, the behavior detection section 101 may detect at least one of a left/right turn and acceleration/deceleration of the vehicle, by using any one of the vehicle operating states such as steering for a left/right turn, using the turn signal for a left/right turn, braking or engine braking for deceleration, using the hazard lights for a stop, and accelerating for acceleration.

Further, the navigation section 107 includes a general navigation device, i.e., includes: a GPS (Global Positioning System) receiver for acquiring a current position; a memory for storing map information; an operation input section for setting a destination; a route search section for calculating a recommended route from the vehicle's position received by the GPS receiver to an inputted destination and thus for matching the calculated recommended route to a roadmap; and a display section for displaying the recommended route with road information.

The behavior detection section 101 may detect at least one of the behaviors such as a right turn, a left turn, acceleration, and deceleration of the vehicle, also based on information outputted from the navigation section 107. Note that when the navigation section 107 is providing route guidance for the vehicle, the behavior detection section 101 may acquire, from the navigation section 107, road information related to the route of which the guidance is provided by the navigation section 107. Alternatively, when the navigation section 107 is not providing route guidance for the vehicle, the behavior detection section 101 may acquire, through the capture section 108, road information related to the forward traveling direction of the vehicle. Here, the road information acquired from the navigation section 107 by the behavior detection section 101 may include, for example, the angle of a left/right turn, the curvature of a straight road, the inclination angle of a road, a road surface condition, a road width, the presence or absence of traffic lights, one-way traffic, no entry, halt, and/or whether or not the vehicle is traveling a right-turn-only lane or a left-turn-only lane.

Further, the capture section 108 includes a camera so as to capture the periphery of the vehicle, particularly the forward traveling direction of the vehicle.

The behavior detection section 101 may acquire at least one of the behaviors such as a right turn, a left turn, acceleration, and deceleration of the vehicle, also by acquiring the road information related to the forward traveling direction of the vehicle by performing image processing based on image information which is related to an image captured by the capture section 108 and is outputted therefrom. Here, the road information acquired by the behavior detection section 101 performing the image processing is the same as the road information acquired from the navigation section 107 by the behavior detection section 101.

Further, a computer having a CPU (Central Processing Unit), a ROM (Read Only Memory), a RAM (Random Access Memory), and the like may be provided in the vehicle so as to function as the behavior detection section 101.

The background image generation section 102 generates a background image in accordance with the acceleration and/or the angular velocity of the vehicle which are detected by the behavior detection section 101.

The image generation section 103 includes a device for outputting images of a TV, a DVD (Digital Versatile Disk) player, a movie, a game, and the like.

The image transformation section 104 transforms, in accordance with the acceleration and/or the angular velocity of the vehicle which are detected by the behavior detection section 101, an image generated by the image generation section 103. In the present embodiment, the image is reduced.

The composition section 105 makes a composite image of the background image generated by the background image generation section 102 and the image transformed by the image transformation section 104. The composite image is made such that the image transformed by the image transformation section 104 is placed in the foreground and the background image generated by the background image generation section 102 is placed in the background.

The display section 106 includes at least one of a liquid crystal display, a CRT display, an organic electroluminescent display, a plasma display, a projector for displaying an image on a screen, a head-mounted display, a head-up display, and the like.

Further, the display section 106 may be positioned to be viewable by a passenger, not the driver, for example, provided for the back seat of the vehicle or provided at the ceiling of the vehicle. Needless to say, the display section 106 may be positioned to be viewable by the driver, but may be preferably positioned to be viewable by the passenger as a priority.

The background image setting section 109 may be, for example, a keyboard or a touch panel, each for selecting the type of the background image generated by the background image generation section 102.

Further, based on the behavior detected by the behavior detection section 101, the background image setting section 109 sets the degree of changing the display position of the background image generated by the background image generation section 102.

Furthermore, based on the behavior detected by the behavior detection section 101, the background image setting section 109 changes and sets, depending on the display position provided on the display section 106, the degree of changing the display position of the background image.

The image transformation setting section 110 may be, for example, a keyboard or a touch panel, each for setting the image transformation section 104 to perform anyone of a trapezoidal transformation, a reduction, and no transformation on the image to be transformed.

Further, the image transformation setting section 110 sets the shape and the reduction ratio of the trapezoid for the transformation to be performed.

Furthermore, based on the behavior detected by the behavior detection section 101, the image transformation setting section 110 sets the degree of transforming the image.

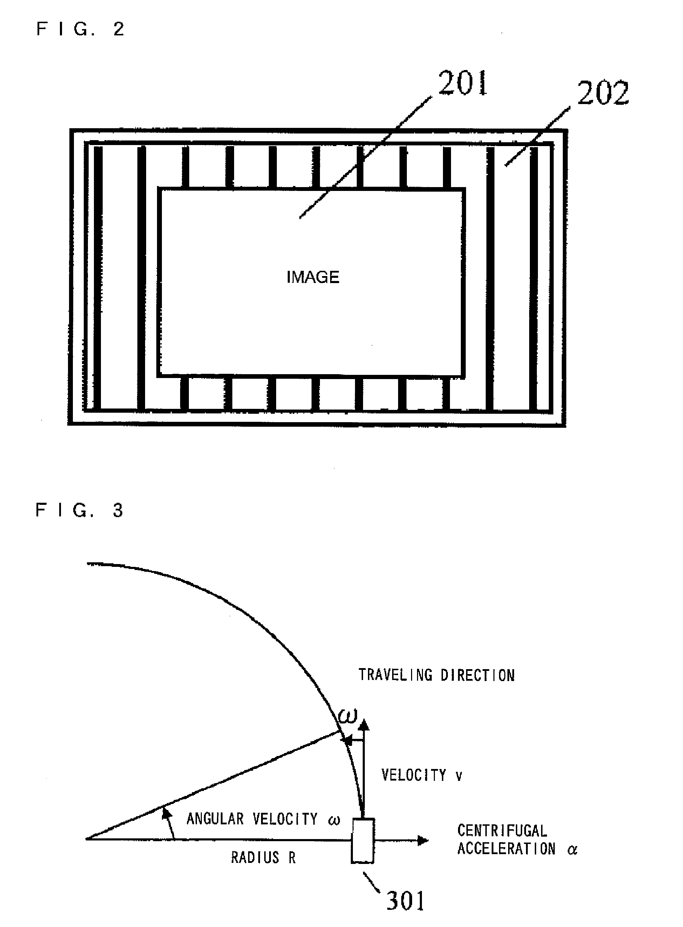

With reference to FIG. 2, the operation of the image display device having the above-described structure will be described. FIG. 2 is an example of display performed by the display section 106 and includes an image 201 and a background image 202. The image 201 is the image reduced by the image transformation section 104 in the case where the image transformation setting section 110 sets the image transformation section 104 to perform the reduction. In this example, the image 201 remains reduced to a constant size, regardless of the behavior outputted from the behavior detection section 101. The image 201 is so reduced as to be easily viewed and also as to allow the background image 202 (a vertical stripe pattern in FIG. 2) to be viewed.

The background image 202 is the background image outputted from the background image generation section 102 in accordance with the behavior detected by the behavior detection section 101, in the case where the background image setting section 109 sets the background image generation section 102 to generate a vertical stripe pattern.

The background image 202 may be the vertical stripe pattern as shown in FIG. 2 or may be a still image such as a photograph. It is only necessary to allow the passenger to recognize that the background image 202 moves when the background image 202 moves. The display position of the background image 202 moves to the left or to the right in accordance with the behavior detected by the behavior detection section 101. In the present embodiment, when the behavior detection section 101 outputs a leftward angular velocity, i.e., when the vehicle turns left, the background image 202 outputted from the background image generation section 102 moves to the right. Note that on the other hand, when the behavior detection section 101 outputs a rightward angular velocity, i.e., when the vehicle turns right, the background image 202 outputted from the background image generation section 102 moves to the left.

Motion sickness is also induced by a visual stimulus. For example, when a person watches a movie featuring intense movements, cinerama sickness occurs. Further, a visual stimulus causes a person self-motion perception of himself/herself rolling, i.e., visually induced self-motion perception (vection). For example, if a rotating drum is rotated with an observer placed in its center, visually induced self-motion perception of starting to feel that he/she himself/herself is rotating in the opposite direction of the rotation of the rotating drum, occurs. The background image may be moved in accordance with the behavior of the vehicle so as to actively give the passenger visually induced self-motion perception, whereby visual information is subconsciously matched to vestibular information obtained from the motion of the vehicle, and particularly matched to a sense of rotation and somatosensory information which are detected by his/her semicircular canals. Thus, it is considered possible to reduce the occurrence of motion sickness more than conventionally provide audio guidance such as "the car will decelerate" or "the car will turn right" and more than display a rightward arrow when the vehicle turns right.

FIG. 3 is a diagram illustrating an angular velocity and a centrifugal acceleration which are generated while a vehicle is traveling along a curve. A vehicle 301 is moving along a curve having a radius R and toward the upper portion of the figure at a velocity v. In this case, an angular velocity $\omega$ can be calculated by an angular velocity sensor which is the behavior detection section 101, and a centrifugal acceleration $\alpha$ can be calculated by an acceleration sensor which is also the behavior detection section 101. In this case, if the moving velocity of the background image outputted from the background image generation section 102 is u, u is represented by a function Func1 of $\omega$ and $\alpha$ as shown in equation 1. Here, the function Func1 can be set by the background image setting section 109.

$$u=Func1(\omega,\alpha) \qquad (equation\ 1)$$

Here, $\alpha$ and w have a relationship of equation 2.

$$\alpha=R\times\omega^2 \qquad (equation\ 2)$$

Note that since the angular velocity $\omega$ and the acceleration $\alpha$ can be measured by the angular velocity sensor and the acceleration sensor, respectively, the radius R can be calculated by equation 3 based on equation 2.

$$R=\alpha/\omega^2 \qquad (equation\ 3)$$

Note that the following relationship holds true.

$$v=R\times\omega \qquad (equation\ 4)$$

Thus, the variable is replaced in equation 1, whereby u can be represented by a function Func2 of $\omega$ and R as shown in equation 5.

$$u=Func2(\omega,R) \qquad (equation\ 5)$$

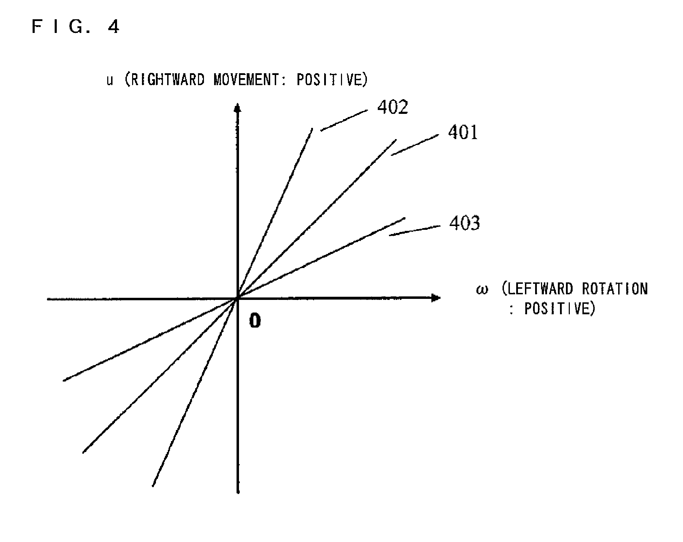

Here, if the radius R is constant, equation 5 is shown in FIG. 4 as a relationship between the angular velocity $\omega$ outputted from the behavior detection section 101 and the moving velocity u of the background image outputted from the background image generation section 102. The positive value of $\omega$ represents the leftward rotation of the vehicle and the negative value of $\omega$ represents the rightward rotation of the vehicle. The positive value of u represents the rightward movement of the background image and the negative value of u represents the leftward movement of the background image. 401 of FIG. 4 indicates that when $\omega$ is great in the positive direction, i.e., when the vehicle rotates to the left, u is great in the positive direction, i.e., the moving velocity of the background image is great in the rightward direction. When $\omega$ is great in the negative direction, i.e., when the vehicle rotates to the right, u is great in the negative direction, i.e., the moving velocity of the background image is great in the leftward direction. 402 is an example where the moving velocity u changes by a large amount with respect to $\omega$, whereas 403 is an example where the moving velocity u changes by a small amount with respect to $\omega$. The above-described relationships can be set by the function Func2 of equation 5. As described above, by the setting of the function Func2, visually induced self-motion perception is caused in accordance with the behavior of the vehicle.

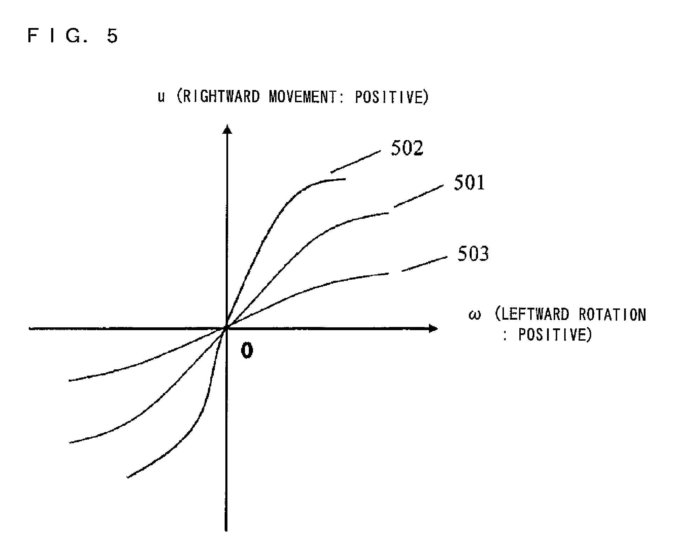

Further, equation 5 can also be represented as shown in FIG. 5. Although the relationship between $\omega$ and u is linear in FIG. 4, 501 indicates that the absolute value of u is saturated when the absolute value of $\omega$ is great. 502 is an example where u changes by a larger amount with respect to $\omega$ than that of 501 does, whereas 503 is an example where u changes by a smaller amount with respect to $\omega$ than that of 501 does. As described above, the relationship between $\omega$ and u is nonlinear in 501, 502, and 503 such that u is saturated at a constant value even when $\omega$ is great. Consequently, even when the vehicle makes a sharp turn and $\omega$ is suddenly increased, the moving velocity u of the background image is maintained at the constant value, and thus the background image cannot become difficult to view. The above-described relationships can be set by the function Func2 of equation 5. As described above, by the setting of the function Func2, visually induced self-motion perception is caused in accordance with the behavior of the vehicle.

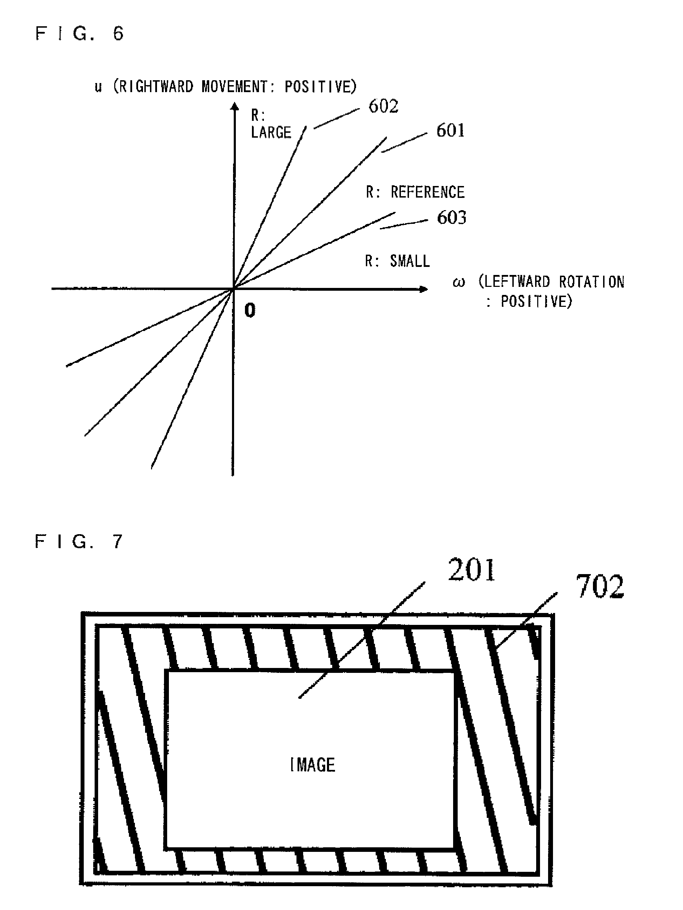

Note that when R changes, $\alpha$ is increased in proportion to R based on equation 2, and thus equation 5 can be represented as shown in FIG. 6. When R of 601 is a reference radius, 602 is an example where the moving velocity u changes by a large amount with respect to $\omega$ since R of 602 is larger than that of 601, whereas 603 is an example where the moving velocity u changes by a small amount with respect to w since R of 603 is smaller than that of 601. The above-described relationships can be set by the function Func2 of equation 5. As described above, by the setting of the function Func2, visually induced self-motion perception is caused in accordance with the behavior of the vehicle. Note that similarly to the case of FIG. 5, the relationship between $\omega$ and u may not be linear such that the absolute value of u is saturated when the absolute value of w is great.

Note that when R changes, the background image 202 of FIG. 2 may be rotated as a background image 702 of FIG. 7, taking into account the effect of the centrifugal acceleration $\alpha$. That is, in accordance with the angular velocity detected by the behavior detection section 101, the background image generation section 102 may generate the background image 702 rotated to the left (i.e., rotated counterclockwise) when the angular velocity indicates a left turn, and may generate the background image 702 rotated to the right (i.e., rotated clockwise) when the angular velocity indicates a right turn. Here, it is set that the greater the value of R, the greater the rotation angle. Note, however, that the rotation angle is limited so as not to make the vertical stripe pattern horizontal. The background image 702 may be rotated while moving at the moving velocity u, or may be rotated only.

Note that if the angular velocity of the movement of the background image outputted from the background image generation section 102 is $\omega 0$ when the distance from the passenger to the display section 106 is L, u can be represented by equation 6, using L and $\omega 0$.

$$u = L \times \omega 0 \qquad \text{(equation 6)}$$

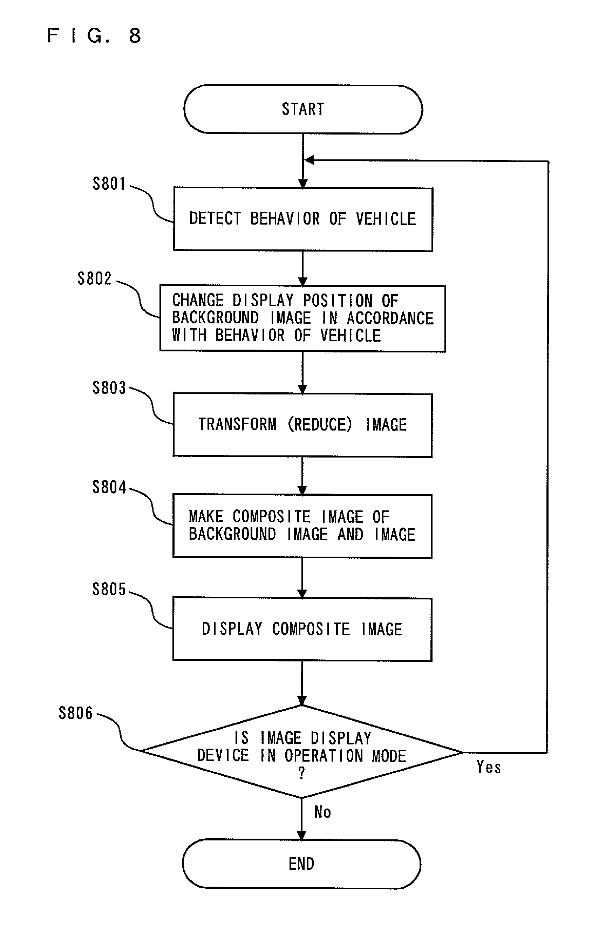

Next, with reference to a flow chart of FIG. 8, the operation of the image display device will be described. First, the behavior detection section 101 detects the current behavior of the vehicle (step S801). For example, the behavior detection section 101 detects at least one of the upward/downward acceleration, the leftward/rightward acceleration, the forward/backward acceleration, and the angular velocity of the vehicle, by using any one of acceleration/deceleration sensed by a velocity sensor, acceleration/deceleration sensed by an acceleration sensor, an angular velocity (pitching, rolling, and yawing) sensed by an angular velocity sensor, and the like.

Next, in accordance with the current behavior of the vehicle which is detected in step S801, the background image generation section 102 changes the display position of a background image based on the setting of the background image setting section 109 (step S802). The moving velocity u of the background image of which the display position is changed is represented by equations 5 and 6, and FIGS. 4, 5 and 6.

Next, the image transformation section 104 transforms an image generated by the image generation section 103 (step S803) . In the present embodiment, it is assumed that the image transformation setting section 110 sets the image transformation section 104 to perform the reduction. Then, the composition section 105 makes a composite image of the background image obtained in step S802 and the image obtained in step S803 (step S804). The composite image is made such that the image transformed by the image transformation section 104 in step S803 is placed in the foreground and the background image generated by the background image generation section 102 in step S802 is placed in the background.

Next, the display section 106 displays the composite image made by the composition section 105 (step S805). Then, it is determined whether or not the image display device is in an operation mode. When the image display device is in the operation mode, the process returns to step S801 and continues. When the image display device is not in the operation mode, the process ends (step S806). Here, the operation mode is the switch as to whether or not a function of the image display device of displaying the background image is available. When the function is not operating, a normal image is to be displayed such that the image is not reduced nor is the background image displayed.

Note that instead of reducing the image outputted from the image transformation section 104, a portion of the image outputted from the image generation section 103 may be clipped and displayed.

Note that the moving velocity u of the background image outputted from the background image generation section 102 is represented by the function of $\omega$ and R in equation 5, but may be viewed as a function of only $\omega$ not including Rby simplifying equation 5.

Note that instead of the background image generation section 102 generating the background image of which the display position is changed, the display position of the background image generated by the background image generation section 102 may remain the same and the display position of the image transformed by the image transformation section 104 may be changed in the composite image made by the composition section 105 and made from the generated background image and the transformed image.

Note that the angular velocity $\omega$ is calculated by the angular velocity sensor which is the behavior detection section 101, but may also be calculated by the navigation section 107. Alternatively, the angular velocity ω may also be calculated by performing image processing on an image of the forward traveling direction captured by the capture section 108.

The effect of the image display device which is confirmed by conducting in-vehicle experiments of the first embodiment of the present invention will be described below.

(Preliminary Experiment 1)

Purpose: when the angular velocity of the movement of the background image 202 outputted from the background image generation section 102 is ω0, to calculate a relationship between a yaw angular velocity ω of the vehicle which is detected by the behavior detection section 101 and ω0, first, the yaw angular velocity w obtained when the vehicle turns at an intersection is measured.

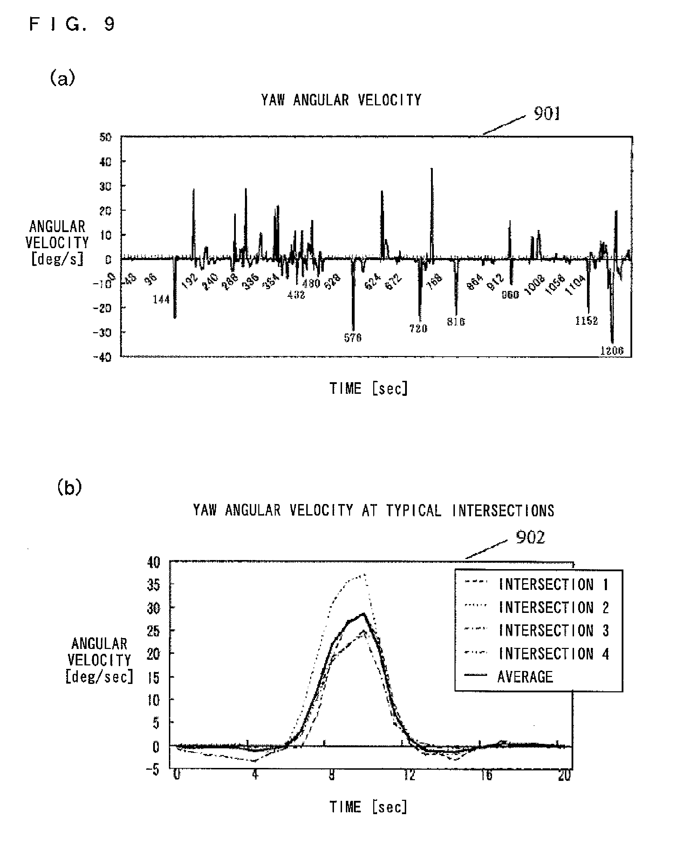

Experimental method: ω is calculated by the angular velocity sensor by traveling a city by car within the speed limit for 20 minutes. Experimental result: the result is shown in FIG. 9. Referring to (a) of FIG. 9, 901 shows the angular velocity obtained during the 20-minute travel. The horizontal axis represents the time and the vertical axis represents the angular velocity. Referring to (b) of FIG. 9, 902 shows typical intersections extracted from the 20-minute travel. The horizontal axis represents the time and the vertical axis represents the angular velocity. The average time it takes to turn at a 90-degree intersection is approximately 6 seconds and the maximum angular velocity is approximately 30 deg/s.

(Preliminary Experiment 2)

Purpose: the relationship between the yaw angular velocity ω of the vehicle which is detected by the behavior detection section 101 and the angular velocity ω0 of the movement of the background image 202 outputted from the background image generation section 102 is calculated.

Experimental method: a Coriolis stimulation device (a rotation device) provided in a dark room of the Faculty of Engineering, Mie University is used. Based on the result of the preliminary experiment 1, a rotation for simulating 902 of (b) of FIG. 9 is generated by the Coriolis stimulation device and the subjects are each rotated by 90 degrees for 6 minutes at up to the maximum angular velocity of 30 deg/s. In accordance with the angular velocity ω [deg/s] generated by the rotation, the background image 202 shown in FIG. 2 is moved in an 11-inch TV at the angular velocity ω0 [deg/s]. The distance between each subject and the display is approximately 50 cm. The subjects each set ω0 sensed by a visual sense to match the angular velocity ω of the Coriolis stimulation device which is sensed by a sense of balance. The subjects are healthy men and women around 20 years old and the number of experimental trials is 40.

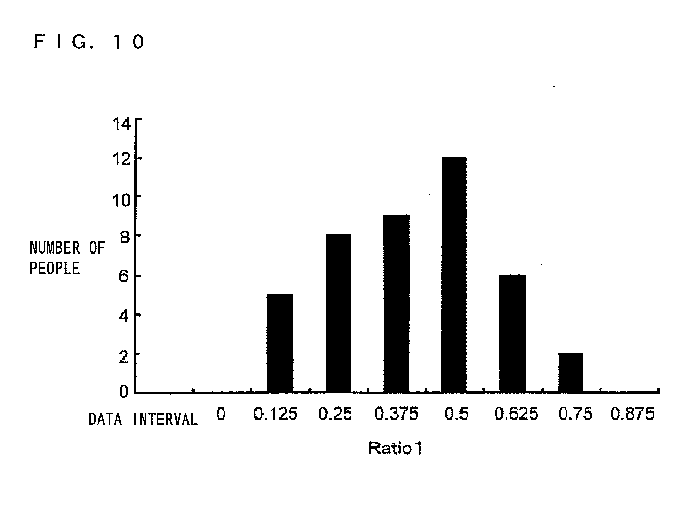

Experimental result: the result is shown in a histogram of FIG. 10. If the ratio between ω0 and ω is Ratio1, Ratio1 is represented by equation 7. The horizontal axis represents Ratio1 and the vertical axis represents the number of the subjects who fall within Ratio1.

$$\mathrm{Ratio1} = \omega 0 / \omega \qquad\qquad (\text{equation } 7)$$

The average value of Ratio1 is 0.47. The standard deviation of Ratio1 is 0.17.

(Actual Experiment 1)

Purpose: the effect of the image display device of the first embodiment of the present invention is confirmed by conducting an in-vehicle experiment.

Experimental method: the in-vehicle experiment is conducted by providing the subjects with a full explanation of the purpose, the procedure, the possible effects, and the like of the experiment and obtaining written prior consent from the subjects. The in-vehicle experiment is conducted by seating the subjects in the second-row seats, the third-row seats, and the fourth-row seats of a ten-seater van having four-row seats. To confirm the effect, comparison is made among three conditions: a normal condition in which the subjects do not view TV; a TV viewing condition in which the subjects view TV; and a first embodiment condition. In the normal condition, no particular restriction or task is imposed. In the TV viewing condition and the first embodiment condition, an 11-inch TV is attached to the headrest of the seat in front of and approximately 60 cm ahead of each subject and the subjects each watch a movie. In the first embodiment condition, the angular velocity ω0 is determined using the result of the preliminary experiment 2. Note that the 11-inch TV has a resolution of 800 horizontal dots and 480 horizontal dots, is 244 mm wide, 138 mm long, and 280 mm diagonal, and displays the image reduced to 205 mm wide and 115 mm long. The riding time is 21 minutes and the vehicle travels a curvy road having no traffic lights.

Motion sickness discomfort is evaluated each minute by subjective evaluation on a rating scale of 11 from 0 (no discomfort) to 10 (extreme discomfort, a tolerable limit). The subjects are healthy men and women around 20 years old and the number of experimental trials is 168: 53 in the normal condition; 53 in the TV viewing condition; and 62 in the first embodiment condition.

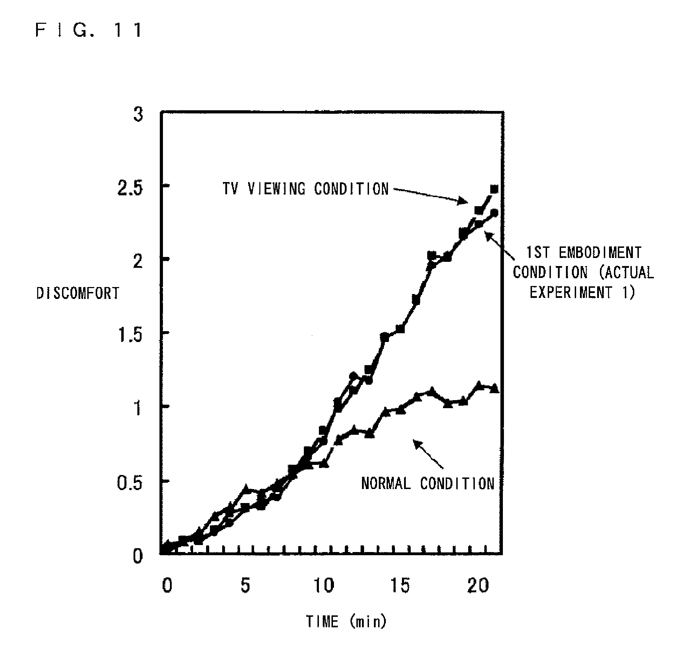

Experimental result: the result is shown in FIG. 11. Since it is confirmed in advance that the rating scale and a distance scale are in proportion to each other, FIG. 11 indicates the average value of the discomfort in each condition. The horizontal axis represents the riding time and the vertical axis represents the discomfort. It is confirmed that the discomfort is far greater in the TV viewing condition than in the normal condition. Additionally, the discomfort is slightly less in the first embodiment condition than in the TV viewing condition.

(Actual Experiment 2)

Purpose: the effect of the image display device of the first embodiment of the present invention is confirmed by conducting an in-vehicle experiment. After the actual experiment 1, a plurality of the subjects are of the opinion that the discomfort is all the more increased since the angular velocity $\omega 0$ of the movement of the background image is great. Therefore, the effect is confirmed by conducting an in-vehicle experiment, with $\omega 0$ reduced.

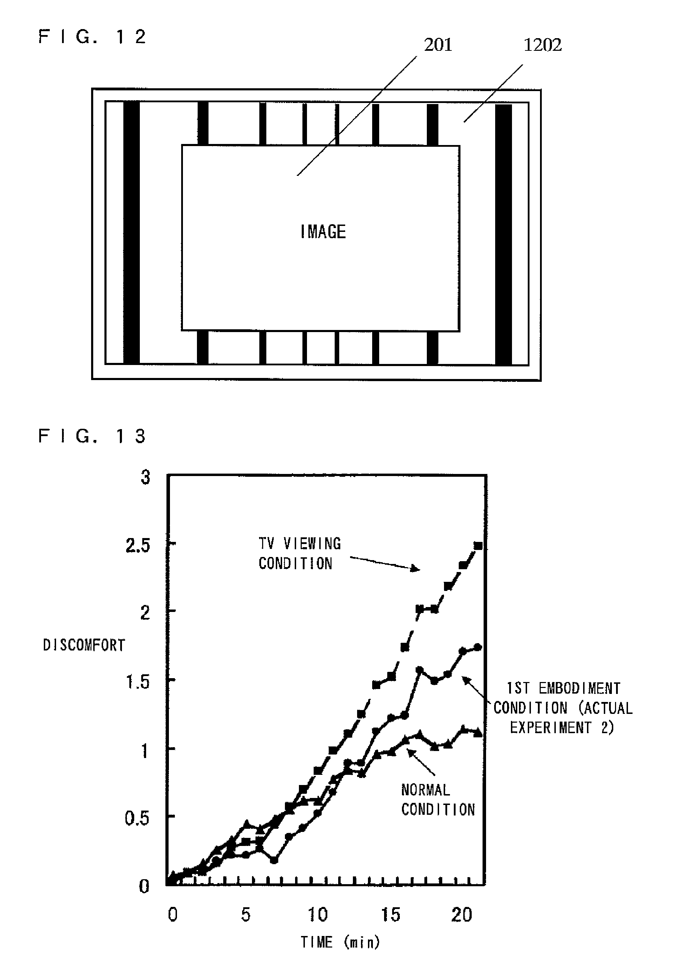

Experimental method: since the subjects each fix their eyes on the image of the TV, the horizontal viewing angle of the image captured by the TV is assumed to correspond to approximately the horizontal viewing angle of an effective field of view. Thus, $\omega 0$ is adjusted to match the angular velocity $\omega$ of the movement of the vehicle when the horizontal viewing angle of the image of the TV is calculated, assumed to be 90 degrees. The adjusted $\omega 0$ is approximately half of that in the actual experiment 1. Further, to create an effect of rotation, a cylindrical effect is provided to the background image outputted from the background image generation section 102. As shown in FIG. 12, a background image 1202 is an image captured from the center of a rotated cylinder having an equally-spaced and equally-wide vertical stripe pattern. As a result, the stripes move quickly in the central portion of the display screen and move slowly at the right and left ends of the display screen. That is, based on the behavior detected by the behavior detection section 101, the background image setting section 109 changes and sets, depending on the display position provided on the display section 106, the degree of changing the display position of the background image. In the actual experiment 2, the number of experimental trials in the first embodiment condition is 24. The other conditions are the same as those of the actual experiment 1.

Experimental result: the result of the actual experiment 1 in the normal condition and the TV viewing condition and of the actual experiment 2 is shown in FIG. 13. Since it is confirmed in advance that the rating scale and the distance scale are in proportion to each other, FIG. 13 indicates the average value of the discomfort in each condition. The horizontal axis represents the riding time and the vertical axis represents the discomfort. It is confirmed that the discomfort is far less in the first embodiment condition (the actual experiment 2) than in the TV viewing condition.

As described above, based on the image display device of the first embodiment of the present invention, the behavior detection section 101 for detecting the behavior of a vehicle, the background image generation section 102 for generating a background image based on the behavior detected by the behavior detection section 101, the image transformation section 104 for transforming an image based on the behavior detected by the behavior detection section 101, the composition section 105 for making a composite image of the background image generated by the background image generation section 102 and the image transformed by the image transformation section 104, and the display section 106 for displaying the composite image made by the composition section 105 are included, whereby it is possible to reduce the burden on a passenger and reduce the occurrence of motion sickness, by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

(Second Embodiment)

FIG. 1 shows an image display device of a second embodiment of the present invention. The second embodiment of the present invention is different from the first embodiment in the operations of the background image setting section 109, the background image generation section 102, the image transformation setting section 110, and the image transformation section 104.

The background image setting section 109 sets the background image generation section 102 to generate the background image in accordance with the acceleration/deceleration or the angular velocity of the vehicle which is detected by the behavior detection section 101. In the present embodiment, the background image setting section 109 sets the background image generation section 102 to generate a black image as the background image. The background image may be a single color image such as a blue screen or may be a still image, instead of the black image.

The image transformation setting section 110 sets the image transformation section 104 to transform, in accordance with the acceleration/deceleration or the angular velocity of the vehicle which is detected by the behavior detection section 101, the image generated by the image generation section 103. In the present embodiment, the image transformation setting section 110 sets the image transformation section 104 to perform the trapezoidal transformation by performing any of an enlargement and a reduction of at least one of the left end, the right end, the top end, and the bottom end of the image in accordance with the behavior of the vehicle. The other elements are the same as those of the first embodiment, and therefore will not be described.

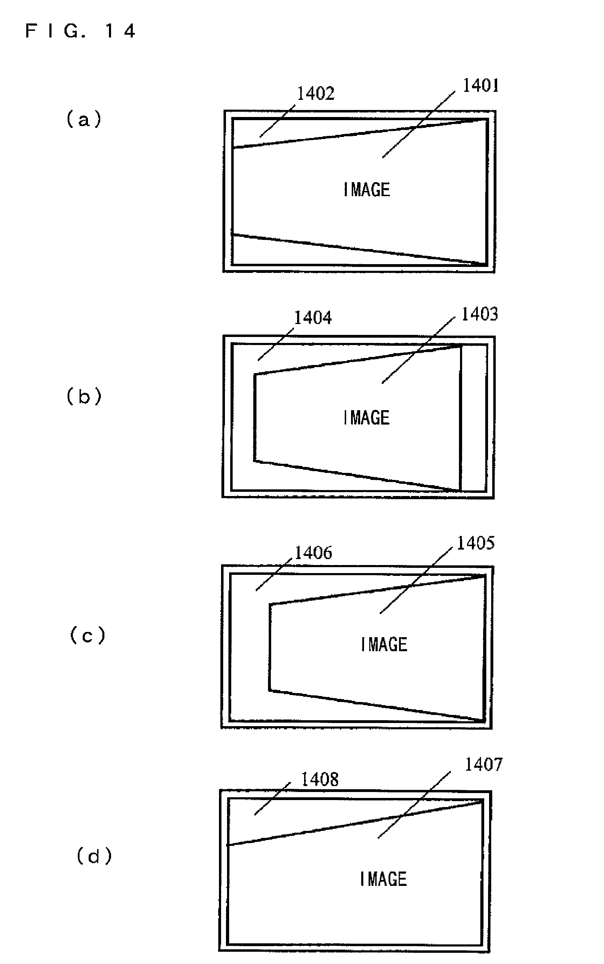

The operation of the image display device having the above-described structure will be described. (a) of FIG. 14 is an example of display performed by the display section 106. An image 1401 is the image trapezoidal-transformed by the image transformation section 104. In this example, the image is trapezoidal-transformed in accordance with the behavior outputted from the behavior detection section 101. In the present embodiment, when the behavior detection section 101 outputs a leftward angular velocity, i.e., when the vehicle turns left, the left end of the image 1401 outputted from the image transformation section 104 is reduced. Note that on the other hand, when the behavior detection section 101 outputs a rightward angular velocity, i.e., when the vehicle turns right, the right end of the image 1401 outputted from the image transformation section 104 is reduced. A background image 1402, which is the background image outputted from the background image generation section 102, may be a single color image such as a black image or a blue screen, or may be a still image.

Note that as another example, (b) of FIG. 14 shows that when the behavior detection section 101 outputs a leftward angular velocity, i.e. , when the vehicle turns left, the left end, the top end, and the bottom end of an image 1403 outputted from the image transformation section 104 are reduced. Note that on the other hand, when the behavior detection section 101 outputs a rightward angular velocity, i.e. , when the vehicle turns right, the right end, the top end, and the bottom end of the image 1403 outputted from the image transformation section 104 are reduced. The image 1403 corresponds to a horizontal rotation of the image around the central axis of the horizontal direction of the image. A background image 1404, which is the background image outputted from the background image generation section 102, may be a single color image such as a black image or a blue screen, or may be a still image.

Note that as another example, (c) of FIG. 14 shows that when the behavior detection section 101 outputs a leftward angular velocity, i.e., when the vehicle turns left, the left end, the top end, and the bottom end of an image 1405 outputted from the image transformation section 104 are reduced, except for the top and bottom ends on the right-end side. Note that on the other hand, when the behavior detection section 101 outputs a rightward angular velocity, i.e., when the vehicle turns right, the right end, the top end, and the bottom end of the image 1405 outputted from the image transformation section 104 are reduced, except for the top and bottom ends on the left-end side. The image 1405 corresponds to a horizontal rotation of the image around the axis of the right end or the left end of the image. A background image 1406, which is the background image outputted from the background image generation section 102, may be a single color image such as a black image or a blue screen, or may be a still image.

Note that referring to (a), (b), and (c) of FIG. 14, the trapezoidal transformation is performed symmetrically in the upward/downward direction. As another example, (d) of FIG. 14 shows that the trapezoidal transformation is performed asymmetrically in the upward/downward direction. (d) of FIG. 14 shows that when the behavior detection section 101 outputs a leftward angular velocity, i.e., when the vehicle turns left, the left end of an image 1407 outputted from the image transformation section 104 is reduced. Note that on the other hand, when the behavior detection section 101 outputs a rightward angular velocity, i.e., when the vehicle turns right, the right end of the image 1407 outputted from the image transformation section 104 is reduced. A background image 1408, which is the background image outputted from the background image generation section 102, may be a single color image such as a black image or a blue screen, or may be a still image.

As described above, the image transformation setting section 110 can set the image transformation section 104 to trapezoidal-transform the image in accordance with the behavior of the vehicle.

Next, the enlargement and the reduction of the left end and the right end of the image trapezoidal-transformed by the image transformation section 104 will be described. It is assumed that a vehicle 301 is moving along a curve having a radius R and toward the upper portion of the figure at a velocity v, as shown in FIG. 3. In this case, an angular velocity $\omega$ can be calculated by an angular velocity sensor which is the behavior detection section 101, and a centrifugal acceleration $\alpha$ can be calculated by an acceleration sensor which is also the behavior detection section 101.

In this case, in the reduction of the left end and the right end of the image trapezoidal-transformed by the image transformation section 104, if the ratio between a left end h1 and a right end h2 is k, k is represented by a function Func3 of $\omega$ and $\alpha$ as shown in equation 8. The function Func3 can be set by the image transformation setting section 110. Note, however, that k is limited to a positive value.

$$k=h2/h1=Func3(\omega,\alpha) \qquad (equation\ 8)$$

Here, $\alpha$ and $\omega$ have a relationship of equation 9.

$$\alpha = R \times \omega^2 \qquad \qquad \text{(equation 9)}$$

Consequently, the variable is replaced in equation 8, whereby k can be represented by a function Func4 of $\omega$ and R as shown in equation 10.

$$k = \text{Func4}(\omega, R) \qquad \qquad \text{(equation 10)}$$

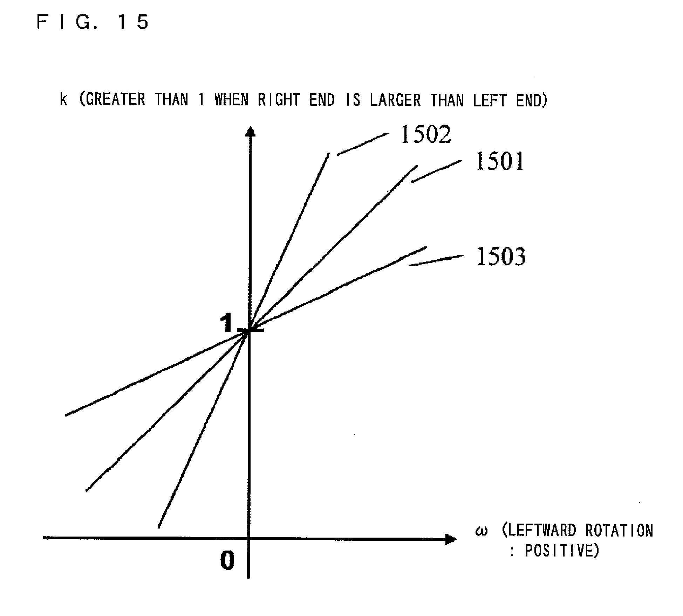

If the radius R is constant, equation 10 is shown in FIG. 15 as a relationship between: the angular velocity $\omega$ outputted from the behavior detection section 101; and the ratio k between the left end h1 and the right end h2 of the image trapezoidal-transformed by the image transformation section 104. The positive value of $\omega$ represents the leftward rotation of the vehicle and the negative value of $\omega$ represents the rightward rotation of the vehicle. k is greater than 1 when the right end h2 is larger than the left end h1, and k is smaller than 1 when the right end h2 is more reduced than the left end h1 is. 1501 of FIG. 15 indicates that when $\omega$ is great in the positive direction, i.e., when the vehicle rotates to the left, k is greater than 1, i.e., the right end h2 is larger than the left end h1. When $\omega$ is great in the negative direction, i.e., when the vehicle rotates to the right, k is smaller than 1, i.e., the right end h2 is more reduced than the left end h1 is. 1502 is an example where k changes by a large amount with respect to $\omega$, whereas 1503 is an example where k changes by a small amount with respect to $\omega$. The above-described relationships can be set by the function Func4 of equation 10. As described above, by the setting of the function Func4, visually induced self-motion perception is caused in accordance with the behavior of the vehicle.

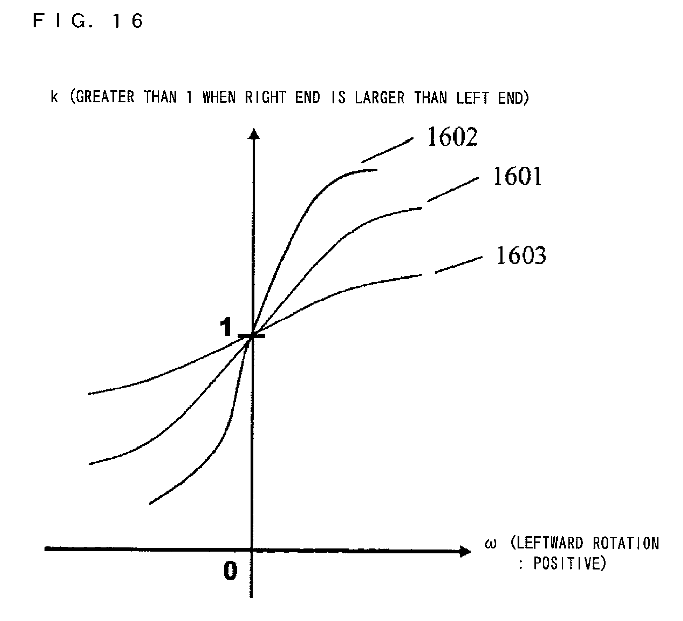

Further, equation 10 can also be represented as shown in FIG. 16. Although the relationship between $\omega$ and k is linear in FIG. 15, 1601 indicates that the absolute value of k is saturated when the absolute value of $\omega$ is great. 1602 is an example where k changes by a larger amount with respect to $\omega$ than that of 1601 does, whereas 1603 is an example where k changes by a smaller amount with respect to $\omega$ than that of 1601 does. As described above, the relationship between $\omega$ and k is nonlinear in 1601, 1602, and 1603 such that k is saturated at a constant value even when $\omega$ is great. Consequently, even when the vehicle makes a sharp turn and $\omega$ is suddenly increased, the ratio k between the left end h1 and the right end h2 is maintained at the constant value, and thus the image cannot become difficult to view. The above-described relationships can be set by the function Func4 of equation 10. As described above, by the setting of the function Func4, visually induced self-motion perception is caused in accordance with the behavior of the vehicle.

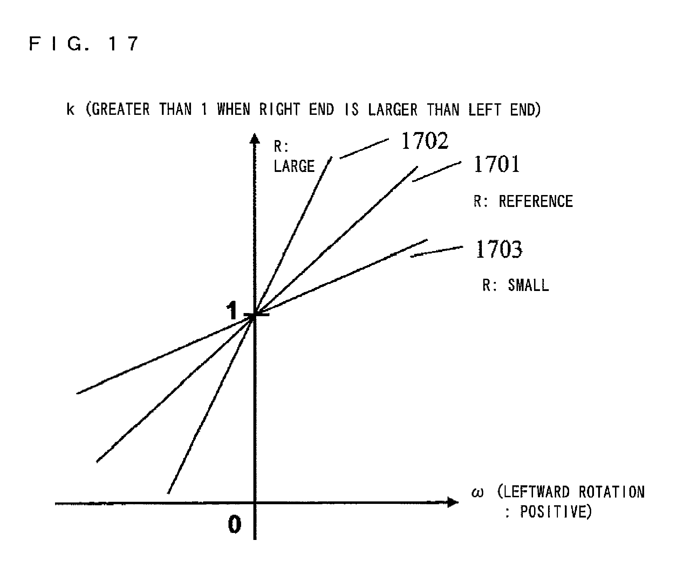

Note that when R changes, $\alpha$ is increased in proportion to R based on equation 9, and thus equation 10 can be represented as shown in FIG. 17. When R of 1701 is a reference radius, 1702 is an example where k changes by a large amount with respect to $\omega$ since R of 1702 is larger than that of 1701, whereas 1703 is an example where k changes by a small amount with respect to $\omega$ since R of 1703 is smaller than that of 1701. The above-described relationships can be set by the function Func4 df equation 10. As described above, by the setting of the function Func4, visually induced self-motion perception is caused in accordance with the behavior of the vehicle. Note that similarly to the case of FIG. 16, the relationship between $\omega$ and k may not be linear such that the absolute value of k is saturated when the absolute value of $\omega$ is great.

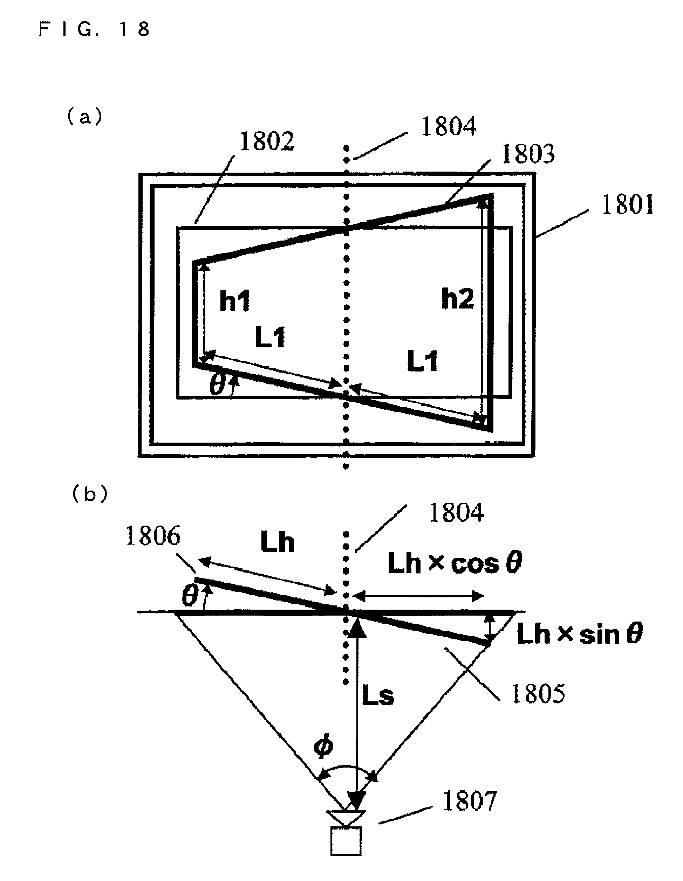

Next, with reference to FIG. 18, the trapezoidal transformation will be described. If a rotation angle related to the trapezoidal transformation performed by the image transformation section 104 is $\theta$, (b) of FIG. 14 can be represented by (a) of FIG. 18. Referring to (a) of FIG. 18, 1801 is the display section 106, and 1802 is the image outputted from the image transformation section 104 in the case where the angular velocity outputted from the behavior detection section 101 is 0, i.e., in the case where the vehicle goes straight. 1803 is the image outputted from the image transformation section 104 in the case where the behavior detection section 101 outputs the leftward angular velocity, i.e., in the case where the vehicle turns left. 1804 represents the central axis of the horizontal direction of the image. In this case, the trapezoidal transformation performed by the image transformation section 104 can be represented by the concept of a virtual camera and a virtual screen both related to computer graphics. That is, as shown in (b) of FIG. 18, if the distance from the virtual camera to the virtual screen is Ls and half the horizontal length of the virtual screen is Lh, equation 10 can be represented by equation 11 when Ls is greater than Lh. Here, 1805 and 1806 are the virtual screen such that 1805 and 1806 correspond to bird's-eye views of the images 1803 and 1802, respectively. 1807 represents the virtual camera. Note that if the horizontal viewing angle of the image captured by the virtual camera is $\varphi$, $\varphi$ can be changed by changing the length of Ls or that of Lh.

$$k=h2/h1=(Ls+Lh \times \sin\theta)/(Ls-Lh \times \sin\theta)$$

$$=(1+Lh/Ls \times \sin\theta)/(1-Lh/Ls \times \sin\theta) \qquad \text{(equation 11)}$$

Here, when a relationship of equation 12 holds true,

$$Lh/Ls \times \sin\theta << 1 \qquad \text{(equation 12)}$$

equation 11 can be approximated to equation 13.

$$k \approx 1+2 \times Lh/Ls \times \sin\theta \qquad \text{(equation 13)}$$

Based on equation 13, FIG. 15 can be represented by the relationship between the angular velocity $\omega$ outputted from the behavior detection section 101 and the rotation angle $\theta$ related to the trapezoidal transformation performed by the image transformation section 104.

Next, the enlargement and the reduction of the top end and the bottom end of the image trapezoidal-transformed by the image transformation section 104 will be described. It is assumed that a vehicle 301 is moving along a curve having a radius R and toward the upper portion of the figure at a velocity v, as shown in FIG. 3. In this case, an angular velocity $\omega$ is calculated by an angular velocity sensor which is the behavior detection section 101. Further, a centrifugal acceleration $\alpha$ is calculated by an acceleration sensor which is also the behavior detection section 101.

In this case, in the trapezoidal transformation performed by the image transformation section 104, if the ratio of the lengths of the top/bottom ends of the image as compared before and after the trapezoidal transformation is m, m is represented by a function Func5 of $\omega$ and $\alpha$ as shown in equation 14. Note, however, that m is limited to a positive value. m = the lengths of the top/bottom ends of the image after the trapezoidal transformation / the lengths of the top/ bottom ends of the image before the trapezoidal transformation

$$=Func5(\omega,\alpha) \qquad \text{(equation 14)}$$

Here, $\alpha$ and $\omega$ have a relationship of equation 15.

$$\alpha = R \times \omega^2 \qquad \text{(equation 15)}$$

Consequently, the variable is replaced in equation 14, whereby m can be represented by a function Func6 of $\omega$ and R as shown in equation 16.

$$m=Func6(\omega,R) \qquad \text{(equation 16)}$$

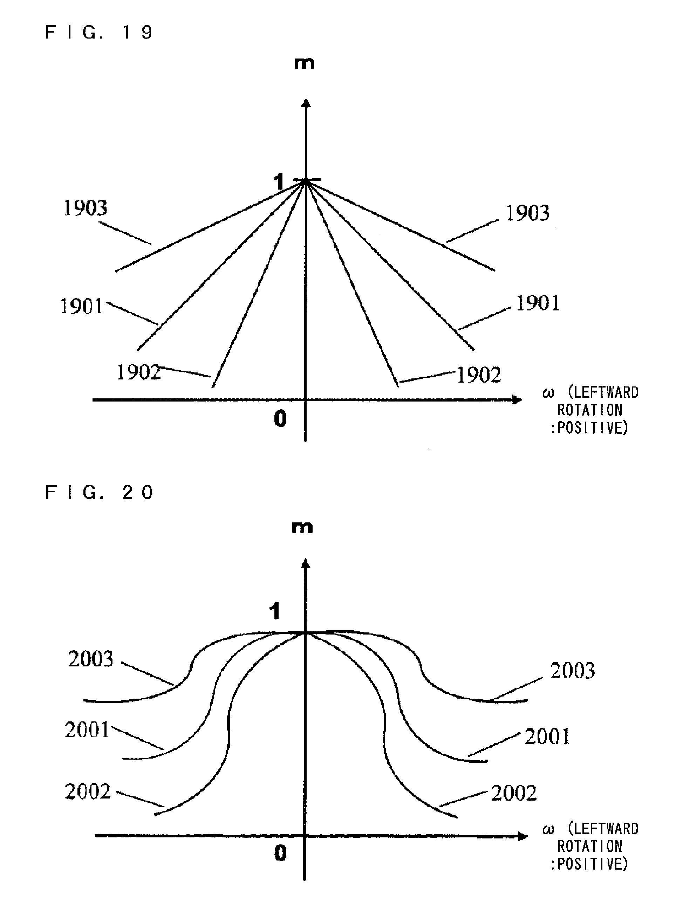

Equation 16 is represented in FIG. 19 as the relationship between: the angular velocity $\omega$ outputted from the behavior detection section 101; and the ratio m of the top/bottom ends of the image as compared before and after the trapezoidal transformation performed by the image transformation section 104. The positive value of $\omega$ represents the leftward rotation of the vehicle and the negative value of $\omega$ represents the rightward rotation of the vehicle, and m is smaller than 1 when the top/bottom ends are reduced. 1901 of FIG. 19 indicates that when $\omega$ is great in the positive direction, i.e., when the vehicle rotates to the left, m is smaller than 1 and the top/bottom ends are reduced. When $\omega$ is great in the negative direction, i.e., when the vehicle rotates to the right, m is smaller than 1 and the top/bottom ends are reduced. 1902 is an example where m changes by a large amount with respect to $\omega$, whereas 1903 is an example where m changes by a small amount with respect to $\omega$. The above-described relationships can be set by the function Func6 of equation 16. As described above, by the setting of the function Func6, visually induced self-motion perception is caused in accordance with the behavior of the vehicle.

Further, equation 16 can also be represented as shown in FIG. 20. Although the relationship between ω and m is linear in FIG. 19, 2001 indicates that the absolute value of m is saturated when the absolute value of ω is great. 2002 is an example where m changes by a larger amount with respect to ω than that of 2001 does, whereas 2003 is an example where m changes by a smaller amount with respect to ω than that of 2001 does. As described above, the relationship between ω and m is nonlinear in 2001, 2002, and 2003 such that m is saturated at a constant value even when ω is great. Consequently, even when the vehicle makes a sharp turn and ω is suddenly increased, the ratio m of the top/bottom ends of the image as compared before and after the trapezoidal transformation is maintained at the constant value, and thus the image cannot become difficult to view. The above-described relationships can be set by the function Func6 of equation 16. By the setting of the function Func6, visually induced self-motion perception is caused in accordance with the behavior of the vehicle.

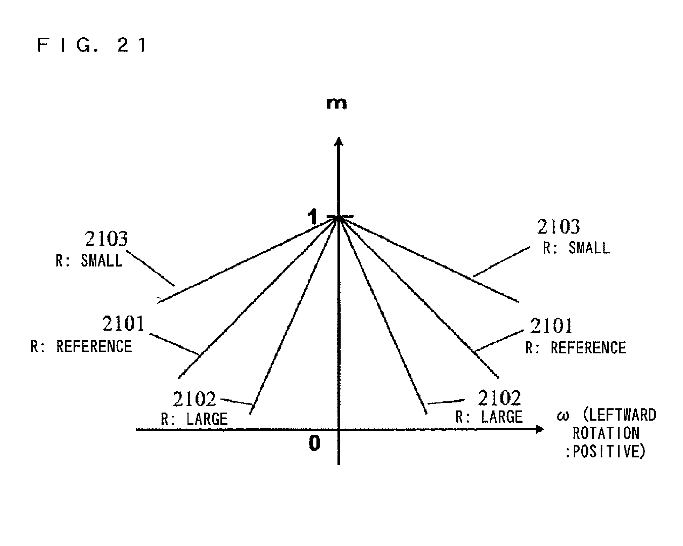

Note that when R changes, α is increased in proportion to R based on equation 15, and thus equation 16 can be represented as shown in FIG. 21. When R of 2101 is a reference radius, 2102 is an example where m changes by a large amount with respect to ω since R of 2102 is larger than that of 2101, whereas 2103 is an example where m changes by a small amount with respect to ω since R of 2103 is smaller than that of 2101. The above-described relationships can be set by the function Func6 of equation 16. As described above, by the setting of the function Func6, visually induced self-motion perception is caused in accordance with the behavior of the vehicle. Note that similarly to the case of FIG. 20, the relationship between w and m may not be linear such that the absolute value of m is saturated when the absolute value of ω is great.

Further, if the state of the trapezoidal transformation is represented by FIG. 18, equation 16 can be represented by equation 17.

$$m=Lh×\cos\theta/Lh=\cos\theta \qquad \text{(equation 17)}$$

Based on equation 17, FIG. 19 can be represented by the relationship between the angular velocity ω outputted from the behavior detection section 101 and the rotation angle θ related to the trapezoidal transformation performed by the image transformation section 104.

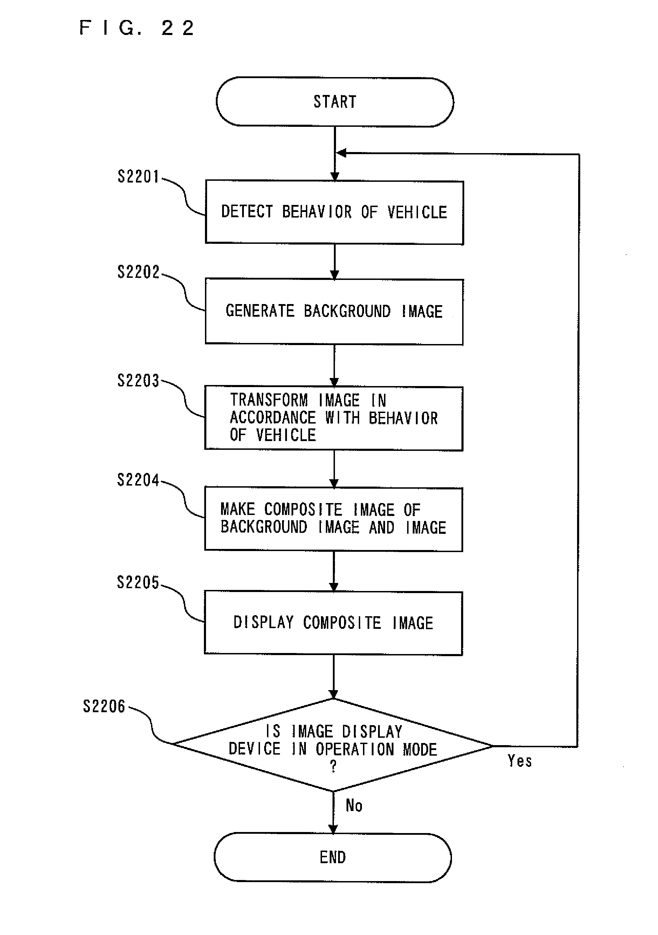

Next, with reference to a flow chart of FIG. 22, the operation of the image display device will be described. Referring to FIG. 22, first, the behavior detection section 101 detects the current behavior of the vehicle (step S2201). For example, the behavior detection section 101 detects at least one of the upward/downward acceleration, the leftward/rightward acceleration, the forward/backward acceleration, and the angular velocity of the vehicle, by using any one of acceleration/deceleration sensed by a velocity sensor, acceleration/deceleration sensed by an acceleration sensor, an angular velocity (pitching, rolling, and yawing), sensed by an angular velocity sensor, and the like.

Next, in accordance with the current behavior of the vehicle which is detected in step S2201, the background image generation section 102 generates a background image based on the setting of the background image setting section 109 (step S2202) . In the present embodiment, the background image may be a single color image such as a black image or a blue screen, or may be a still image.

Next, in accordance with the acceleration/deceleration or the angular velocity of the vehicle which is detected by the behavior detection section 101, the image transformation section 104 transforms an image generated by the image generation section 103 (step S2203). In the present embodiment, based on the setting of the image transformation setting section 110, the image transformation section 104 performs the trapezoidal transformation by performing any of an enlargement and a reduction of at least one of the left end, the right end, the top end, and the bottom end of the image in accordance with the behavior of the vehicle.

Then, the composition section 105 makes a composite image of the background image obtained in step S2202 and the image obtained in step S2203. The composite image is made such that the image transformed by the image transformation section 104 in step S2203 is placed in the foreground and the background image generated by the background image generation section 102 in step S2202 is placed in the background (step S2204).

Next, the composite image made by the composition section 105 is displayed (step S2205). Then, it is determined whether or not the image display device is in an operation mode. When the image display device is in the operation mode, the process returns to step S2201 and continues. When the image display device is not in the operation mode, the process ends (step S2206). Here, the operation mode is the switch as to whether or not a function of the image display device of transforming the image is available. when the function is not operating, a normal image is to be displayed such that the image is not transformed.

Note that when transforming the image, the image transformation section 104 may trapezoidal-transform the image slightly reduced in advance, so as to display the whole area of the image. In this case, one of the left and right ends of the image may be enlarged.

Note that in the trapezoidal transformation performed by the image transformation section 104, the ratio k between the left end and the right end of the trapezoidal-transformed image is represented by the function of ω and R in equation 10, but may be viewed as a function of only ω not including R by simplifying equation 10.

Note that in the trapezoidal transformation performed by the image transformation section 104, the ratio m of the lengths of the top/bottom ends of the image as compared before and after the trapezoidal-transformation is represented by the function of ω and R in equation 16, but may be viewed as a function of only ω not including R by simplifying equation 16.

Note that the angular velocity ω is calculated by the angular velocity sensor which is the behavior detection section 101, but may be calculated by the navigation section 107. Alternatively, the angular velocity ω may be calculated by performing image processing on an image of the forward traveling direction captured by the capture section 108.

The effect of the image display device which is confirmed by conducting an in-vehicle experiment of the second embodiment of the present invention will be described below.

(Preliminary Experiment 1)

Purpose: when the rotation angle related to the trapezoidal transformation performed by the image transformation section 104 is θ, to calculate a relationship between a yaw angular velocity ω of the vehicle which is detected by the behavior detection section 101 and θ, first, the yaw angular velocity ω obtained when the vehicle turns at an intersection is measured.

Experimental method: ω is calculated by the angular velocity sensor by traveling a city by car within the speed limit for 20 minutes. The experimental method is the same as that of the preliminary experiment 1 of the first embodiment of the present invention.

Experimental result: the result is shown in FIG. 9. The result is the same as that of the preliminary experiment 1 of the first embodiment of the present invention.

(Preliminary Experiment 2)

Purpose: the relationship between the yaw angular velocity ω of the vehicle which is detected by the behavior detection section 101 and the rotation angle θ related to the trapezoidal transformation performed by the image transformation section 104 is calculated.

Experimental method: a Coriolis stimulation device (a rotation device) provided in a dark room of the Faculty of Engineering, Mie University is used. Based on the result of the preliminary experiment 1, a rotation for simulating 902 of (b) of FIG. 9 is generated by the Coriolis stimulation device and the subjects are each rotated by 90 degrees for 6 minutes at up to the maximum angular velocity of 30 deg/s. In accordance with the angular velocity ω [deg/s] generated by the rotation, the image 1803 shown in FIG. 18 is trapezoidal-transformed by being rotated by the rotation angle θ [deg] in an 11-inch TV. The distance between each subject and the display is approximately 50 cm. The subjects each set the rotation angle θ sensed by a visual sense to match the angular velocity ω of the Coriolis stimulation device which is sensed by a sense of balance. The subjects are healthy men and women around 20 years old and the number of experimental trials is 40.

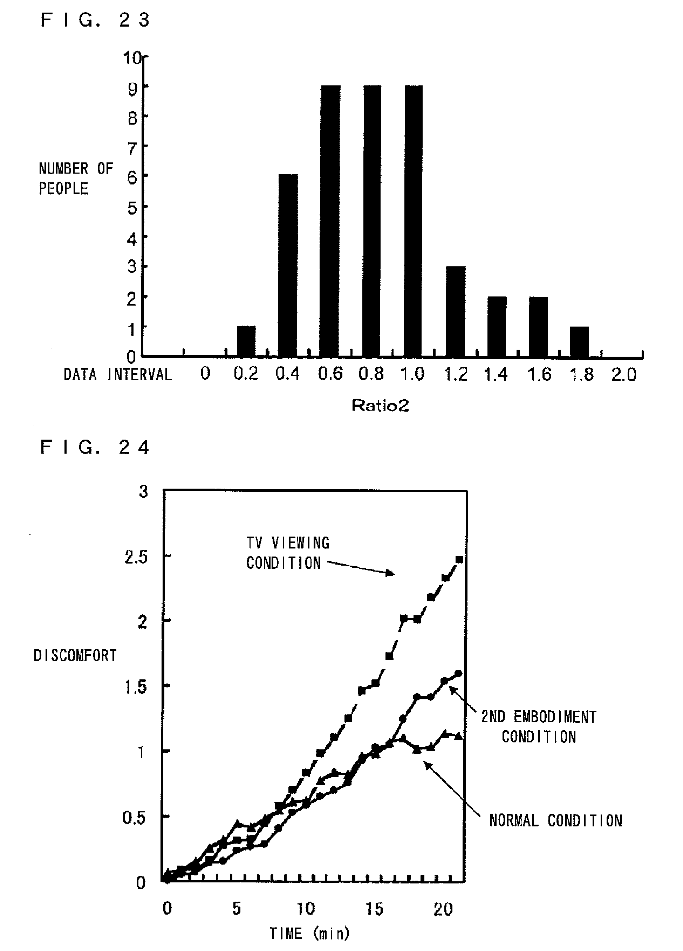

Experimental result: the result is shown in a histogram of FIG. 23. If the ratio between θ and ω is Ratio2, Ratio2 is represented by equation 18. The horizontal axis represents Ratio2 and the vertical axis represents the number of the subjects who fall within Ratio2.

$$\mathtt{Ratio2} = \theta/\omega \qquad\qquad \text{(equation 18)}$$

The average value of Ratio2 is 0.94. The standard deviation of Ratio2 is 0.36.

(Actual Experiment)

Purpose: the effect of the image display device of the second embodiment of the present invention is confirmed by conducting an in-vehicle experiment.

Experimental method: the in-vehicle experiment is conducted by providing the subjects with a full explanation of the purpose, the procedure, the possible effects, and the like of the experiment and obtaining written prior consent from the subjects. The in-vehicle experiment is conducted by seating the subjects in the second-row seats, the third-row seats, and the fourth-row seats of a ten-seater van having four-row seats. To confirm the effect, comparison is made among three conditions: a normal condition in which the subjects do not view TV; a TV viewing condition in which the subjects view TV; and a second embodiment condition. The normal condition and the TV viewing condition are the same as the normal condition and the TV viewing condition, respectively, of the actual experiment 1 of the first embodiment of the present invention. In the second embodiment condition, an 11-inch TV is attached to the headrest of the seat in front of and approximately 60 cm ahead of each subject and the subj ects each watch a movie. In the second embodiment condition, the angle θ is determined using the result of the preliminary experiment 2. Note that the 11-inch TV has a resolution of 800 horizontal dots and 480 horizontal dots, is 244 mm wide, 138 mm long, and 280 mm diagonal, and displays the image reduced to 205 mm wide and 115 mm long. The riding time is 21 minutes and the vehicle travels a curvy road having no traffic lights.

Motion sickness discomfort is evaluated each minute by subjective evaluation on a rating scale of 11 from 0 (no discomfort) to 10 (extreme discomfort, a tolerable limit). The subjects are healthy men and women around 20 years old and the number of experimental trials is 66 in the second embodiment condition.

Experimental result: the result is shown in FIG. 24. Since it is confirmed in advance that the rating scale and a distance scale are in proportion to each other, FIG. 24 indicates the average value of the discomfort in each condition. The horizontal axis represents the riding time and the vertical axis represents the discomfort. It is confirmed that the discomfort is far greater in the TV viewing condition than in the normal condition. Additionally, the discomfort is far less in the second embodiment condition than in the TV viewing condition. Note that although the experiments are conducted in the cases of φ of approximately 30 deg and φ of approximately 60 deg, the discomfort is hardly affected by φ.

As described above, based on the image display device of the second embodiment of the present invention, the behavior detection section 101 for detecting the behavior of a vehicle, the background image generation section 102 for generating a background image based on the behavior detected by the behavior detection section 101, the image transformation section 104 for transforming an image based on the behavior detected by the behavior detection section 101, the composition section 105 for making a composite image of the background image generated by the background image generation section 102 and the image transformed by the image transformation section 104, and the display section 106 for displaying the composite image made by the composition section 105 are included, whereby it is possible to reduce the burden on a passenger and reduce the occurrence of motion sickness, by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

Note that in the present embodiment, the background image generation section 102 generates the background image of a single color image such as a black image or a blue screen or of a still image, such that the composition section 105 makes the composite image of the generated background image and the image transformed by the image transformation section 104. However, it may not be necessary to generate the background image to make the composite image of the generated background image and the transformed image, and the background image generation section 102, the background image setting section 109, and the composition section 105 may not be provided. In this case, an output from the image transformation section 104 is directly inputted to the display section 106. That is, the image display device in this case has a similar effect by including a behavior detection section for detecting the behavior of a vehicle, an image transformation section for transforming an image based on the behavior detected by the behavior detection section, and a display section for displaying the image transformed by the image transformation section.

(Third Embodiment)

FIG. 1 shows an image display device of a third embodiment of the present invention. The third embodiment of the present invention is different from the first embodiment and the second embodiment in the operations of the background image setting section 109, the background image generation section 102, the image transformation setting section 110, and the image transformation section 104.

The background image setting section 109 sets the background image generation section 102 to generate the background image in accordance with the acceleration/deceleration or the angular velocity of the vehicle which is detected by the behavior detection section 101. In the present embodiment, the background image setting section 109 sets the background image generation section 102 to generate a vertical stripe pattern as the background image.

That is, the operations of the background image setting section 109 and the background image generation section 102 of the present embodiment are the same as the operations of the background image setting section 109 and the background image generation section 102, respectively, of the first embodiment.

The image transformation setting section 110 sets the image transformation section 104 to transform, in accordance with the acceleration/deceleration or the angular velocity of the vehicle which is detected by the behavior detection section 101, the image generated by the image generation section 103. In the present embodiment, the image transformation setting section 110 sets the image transformation section 104 to perform the trapezoidal transformation by performing any of an enlargement and a reduction of at least one of the left end, the right end, the top end, and the bottom end of the image in accordance with the behavior of the vehicle.

That is, the operations of the image transformation setting section 110 and the image transformation section 104 of the present embodiment are the same as the operations of the image transformation setting section 110 and the image transformation section 104, respectively, of the second embodiment. The other elements are the same as those of the first embodiment and the second embodiment, and therefore will not be described.

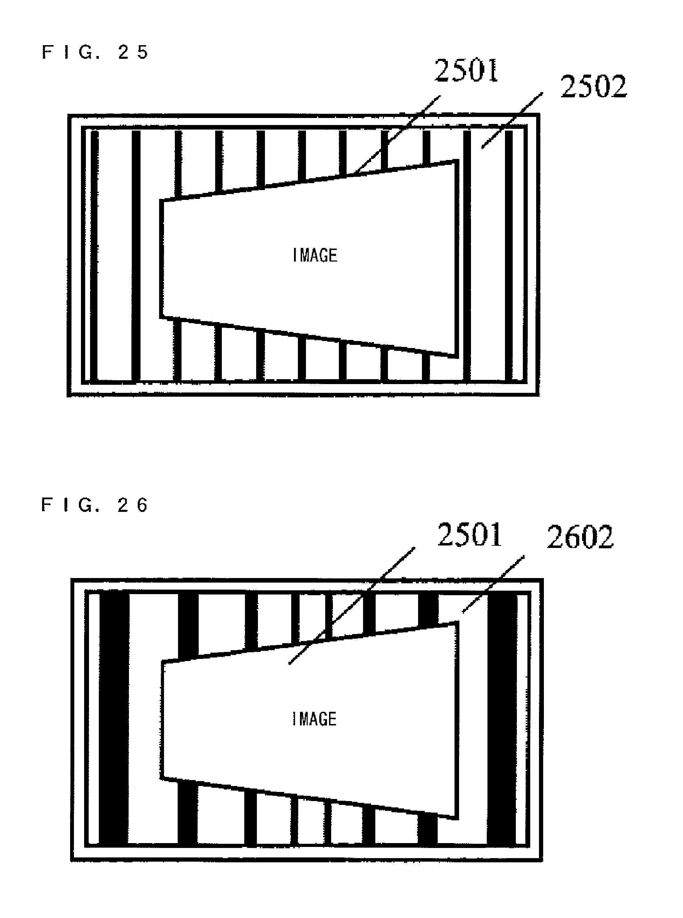

The operation of the image display device having the above-described structure will be described. FIG. 25 is an example of display performed by the display section 106. An image 2501 is the image trapezoidal-transformed by the image transformation section 104. In this example, the image is trapezoidal-transformed in accordance with the behavior outputted from the behavior detection section 101. In the present embodiment, when the behavior detection section 101 outputs a leftward angular velocity, i.e., when the vehicle turns left, the left end, the top end, and the bottom end of the image 2501 outputted from the image transformation section 104 are reduced. Note that on the other hand, when the behavior detection section 101 outputs a rightward angular velocity, i.e., when the vehicle turns right, the right end, the top end, and the bottom end of the image 2501 outputted from the image transformation section 104 are reduced. The image 2501 corresponds to a horizontal rotation of the image around the central axis of the horizontal direction of the image.

The background image 2502 is the background image outputted from the background image generation section 102 in accordance with the behavior detected by the behavior detection section 101, in the case where the background image setting section 109 sets the background image generation section 102 to generate the vertical stripe pattern. The background image 2502 may be the vertical stripe pattern as shown in FIG. 25 or may be a still image such as a photograph. It is only necessary to allow the passenger to recognize that the background image 2502 moves when the background image 2502 moves. The display position of the background image 2502 moves to the left or to the right in accordance with the behavior detected by the behavior detection section 101. In the present embodiment, when the behavior detection section 101 outputs a leftward angular velocity, i.e., when the vehicle turns left, the background image 2502 outputted from the background image generation section 102 moves to the right. Note that on the other hand, when the behavior detection section 101 outputs a rightward angular velocity, i.e., when the vehicle turns right, the background image 2502 outputted from the background image generation section 102 moves to the left.

Further, as an example of display, the vertical stripe pattern set by the background image setting section 102 may be a background image 2602 as shown in FIG. 26. To create an effect of rotation for the background image 2602, a cylindrical effect is provided to the background image outputted from the background image generation section 102. The background image 2602 is an image captured from the center of a rotated cylinder having an equally-spaced and equally-wide vertical stripe pattern.

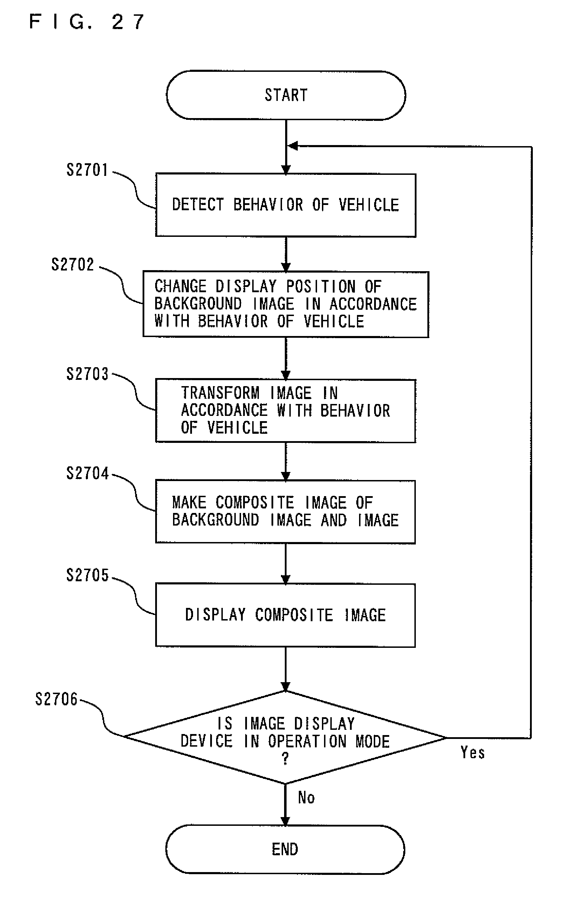

Next, with reference to a flow chart of FIG. 27, the operation of the image display device will be described. Referring to FIG. 27, first, the behavior detection section 101 detects the current behavior of the vehicle (step S2701). For example, the behavior detection section 101 detects at least one of the upward/downward acceleration, the leftward/ rightward acceleration, the forward/backward acceleration, and the angular velocity of the vehicle, by using any one of acceleration/deceleration sensed by a velocity sensor, acceleration/deceleration sensed by an acceleration sensor, an angular velocity (pitching, rolling, and yawing) sensed by an angular velocity sensor, and the like.

Next, in accordance with the current behavior of the vehicle which is detected in step S2701, the background image generation section 102 changes the display position of a background image based on the setting of the background image setting section 109 (step S2702).

Next, the image transformation section 104 transforms, in accordance with the acceleration/deceleration or the angular velocity of the vehicle which is detected by the behavior detection section 101, an image generated by the image generation section 103 (step S2703). In the present embodiment, based on the setting of the image transformation setting section 110, the image transformation section 104 performs the trapezoidal transformation by performing any of an enlargement and a reduction of at least one of the left end, the right end, the top end, and the bottom end of the image in accordance with the behavior of the vehicle.

Then, the composition section 105 makes a composite image of the background image obtained in step S2702 and the image obtained in step S2703. The composite image is made such that the image transformed by the image transformation section 104 in step S2703 is placed in the foreground and the background image generated by the background image generation section 102 in step S2702 is placed in the background (step S2704).

Next, the display section 106 displays the composite image made by the composition section 105 (step S2705). Then, it is determined whether or not the image display device is in an operation mode. When the image display device is in the operation mode, the process returns to step S2701 and continues. When the image display device is not in the operation mode, the process ends (step S2706). Here, the operation mode is the switch as to whether or not functions of the image display device of transforming the image and of displaying the background image are available. When the functions are not operating, a normal image is to be displayed such that the image is not reduced nor is the background image displayed.

The present embodiment is aimed at a synergistic effect between the first embodiment and the second embodiment.

The effect of the image display device which is confirmed by conducting in-vehicle experiments of the third embodiment of the present invention will be described below. As preliminary experiments, the results of the preliminary experiment 1 and the preliminary experiment 2 of the first embodiment and the results of the preliminary experiment 1 and the preliminary experiment 2 of the second embodiment are used.

(Actual Experiment 1)

Purpose: the effect of the image display device of the third embodiment of the present invention is confirmed by conducting an in-vehicle experiment.

Experimental method: the in-vehicle experiment is conducted by providing the subjects with a full explanation of the purpose, the procedure, the possible effects, and the like of the experiment and obtaining written prior consent from the subjects. The in-vehicle experiment is conducted by seating the subjects in the second-row seats, the third-row seats, and the fourth-row seats of a ten-seater van having four-row seats. To confirm the effect, comparison is made among three conditions: a normal condition in which the subjects do not view TV; a TV viewing condition in which the subjects view TV; and a third embodiment condition. The normal condition and the TV viewing condition are the same as the normal condition and the TV viewing condition, respectively, of the actual experiment 1 of the first embodiment of the present invention. In the third embodiment condition, an 11-inch TV is attached to the headrest of the seat in front of and approximately 60 cm ahead of each subject and the subjects each watch a movie. In the third embodiment condition, the angle $\theta$ is determined using the result of the preliminary experiment 2 of the second embodiment. Further, $\omega 0$ is determined using the result of the actual experiment 1 of the first embodiment. Note that the 11-inch TV has a resolution of 800 horizontal dots and 480 horizontal dots, is 244 mm wide, 138 mm long, and 280 mm diagonal, and displays the image reduced to 205 mm wide and 115 mm long. The riding time is 21 minutes and the vehicle travels a curvy road having no traffic lights.

Motion sickness discomfort is evaluated each minute by subjective evaluation on a rating scale of 11 from 0 (no discomfort) to 10 (extreme discomfort, a tolerable limit). The subjects are healthy men and women around 20 years old and the number of experimental trials is 67 in the third embodiment condition.

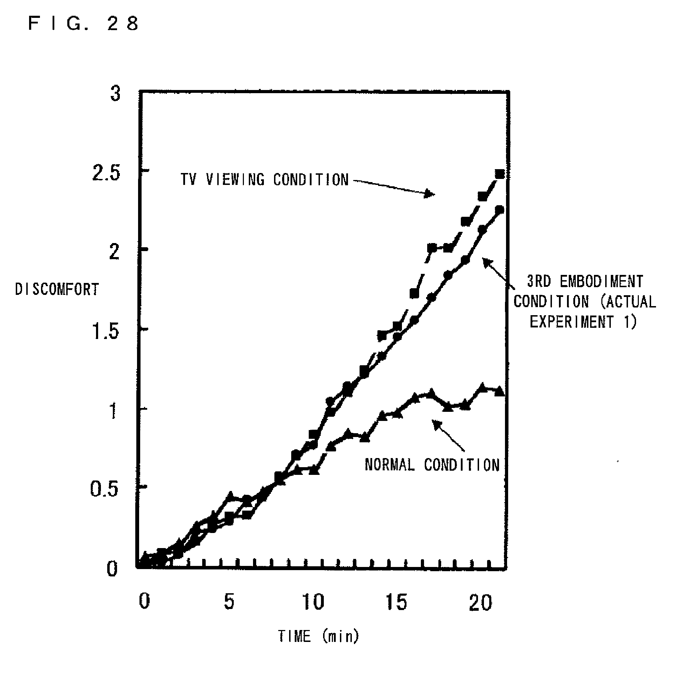

Experimental result: the result is shown in FIG. 28. Since it is confirmed in advance that the rating scale and a distance scale are in proportion to each other, FIG. 28 indicates the average value of the discomfort in each condition. The horizontal axis represents the riding time and the vertical axis represents the discomfort. It is confirmed that the discomfort is far greater in the TV viewing condition than in the normal condition. Additionally, the discomfort is slightly less in the third embodiment condition than in the TV viewing condition. Moreover, it is confirmed that the discomfort is slightly less in the third embodiment condition than in the first embodiment condition (the actual experiment 1).

(Actual Experiment 2)

Purpose: the effect of the image display device of the third embodiment of the present invention is confirmed by conducting an in-vehicle experiment. After the actual experiment 1, a plurality of the subjects are of the opinion that the discomfort is all the more increased since the angular velocity $\omega 0$ of the movement of the background image is great. Therefore, the effect is confirmed by conducting the in-vehicle experiment, with $\omega 0$ reduced.

Experimental method: the in-vehicle experiment is conducted by providing the subjects with a full explanation of the purpose, the procedure, the possible effects, and the like of the experiment and obtaining written prior consent from the subjects. The in-vehicle experiment is conducted by seating the subjects in the second-row seats, the third-row seats, and the fourth-row seats of a ten-seater van having four-row seats. To confirm the effect, comparison is made among three conditions: a normal condition in which the subjects do not view TV; a TV viewing condition in which the subjects view TV; and a third embodiment condition (an actual experiment 2). The normal condition and the TV viewing condition are the same as the normal condition and the TV viewing condition, respectively, of the actual experiment 1 of the first embodiment of the present invention. In the third embodiment condition (the actual experiment 2), an 11-inch TV is attached to the headrest of the seat in front of and approximately 60 cm ahead of each subject and the subjects each watch a movie. In the third embodiment condition (the actual experiment 2), the angle $\theta$ is determined using the result of the preliminary experiment 2 of the second embodiment. Further, $\omega 0$ is determined using the result of the actual experiment 2 of the first embodiment. Furthermore, similarly to the actual experiment 2 of the first embodiment, to create an effect of rotation, a cylindrical effect is provided to the background image outputted from the background image generation section 102. The riding time is 21 minutes and the vehicle travels a curvy road having no traffic lights.

Motion sickness discomfort is evaluated each minute by subjective evaluation on a rating scale of 11 from 0 (no discomfort) to 10 (extreme discomfort, a tolerable limit). The subjects are healthy men and women around 20 years old and the number of experimental trial in the third embodiment condition (the actual experiment 2) is 23.

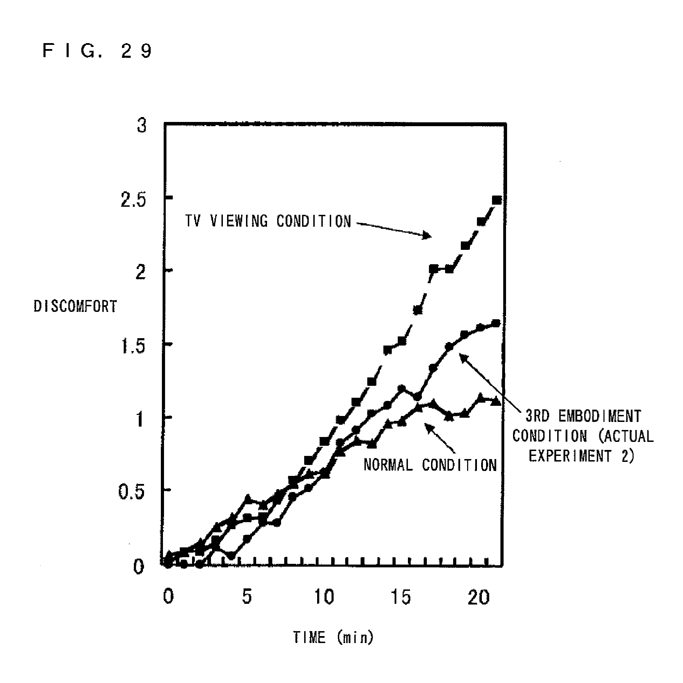

Experimental result: the result is shown in FIG. 29. Since it is confirmed in advance that the rating scale and a distance scale are in proportion to each other, FIG. 29 indicates the average value of the discomfort in each condition. The horizontal axis represents the riding time and the vertical axis represents the discomfort. It is confirmed that the discomfort is far greater in the TV viewing condition than in the normal condition.

Additionally, the discomfort is far less in the third embodiment condition (the actual experiment 2) than in the TV viewing condition. Moreover, it is confirmed that the discomfort is slightly less in the third embodiment condition (the actual experiment 2) than in the first embodiment condition (the actual experiment 2).

As described above, based on the image display device of the third embodiment of the present invention, the behavior detection section 101 for detecting the behavior of a vehicle, the background image generation section 102 for generating a background image based on the behavior detected by the behavior detection section 101, the image transformation section 104 for transforming an image based on the behavior detected by the behavior detection section 101, the composition section 105 for making a composite image of the background image generated by the background image generation section 102 and the image transformed by the image transformation section 104, the display section 106 for displaying the composite image made by the composition section 105 are included, whereby it is possible to reduce the burden on a passenger and reduce the occurrence of motion sickness, by giving the passenger, through a visual sense, perception (visually induced self-motion perception) of his/her own body moving while viewing an image of a TV and the like in the vehicle, and thus by matching visual information to vestibular information obtained from the motion of the vehicle, particularly to a sense of rotation and somatosensory information which are detected by his/her semicircular canals.

The structures described in the foregoing embodiments are merely illustrative and not restrictive. An arbitrary structure can be applied within the scope of the present invention.

INDUSTRIAL APPLICABILITY

As described above, the image display device of the present invention is capable of reducing the burden on a passenger by giving the passenger, through a visual sense, perception (visually induced self-motion perception) of his/her own body moving while viewing an image of a TV and the like in a vehicle, and thus by matching visual information to vestibular information obtained from the motion of the vehicle, particularly to a sense of rotation and somatosensory information which are detected by his/her semicircular canals, and therefore is useful for an anti-motion sickness device and the like which prevent a passenger from suffering from motion sickness.

**Claims**

1. An image display device comprising:

    a behavior detection section for detecting a behavior of a vehicle;
    a background image generation section for generating a background image based on the behavior detected by the behavior detection section;
    an image transformation section for transforming an image based on the behavior of the vehicle which is detected by the behavior detection section;
    a composition section for making a composite image of the background image generated by the background image generation section and the image transformed by the image transformation section; and
    a display section for displaying the composite image made by the composition section.

2. The image display device according to claim 1,
    wherein the behavior detection section detects the behavior of the vehicle, using at least one of signals of a velocity sensor, an acceleration sensor, and an angular velocity sensor.

3. The image display device according to claim 1,
    wherein the behavior detection section detects the behavior of the vehicle based on a state of an operation performed on the vehicle by a driver of the vehicle.

4. The image display device according to claim 1,
    wherein the behavior detection section detects the behavior of the vehicle based on an output from a capture section for capturing an external environment of the vehicle.

5. The image display device according to claim 1,

wherein the behavior detection section detects the behavior of the vehicle based on an output from a navigation section for providing route guidance for the vehicle.

6. The image display device according to claim 1,
wherein the behavior detection section detects one or more of a leftward/rightward acceleration, an upward/downward acceleration, a forward/backward acceleration, and an angular velocity of the vehicle.

7. The image display device according to claim 1,
wherein the background image generation section changes a display position of the background image in accordance with the behavior of the vehicle which is detected by the behavior detection section.

8. The image display device according to claim 1 or claim 7,
wherein in accordance with the behavior of the vehicle which is detected by the behavior detection section, the background image generation section generates the background image moved to the right when the behavior indicates a left turn and also generates the background image moved to the left when the behavior indicates a right turn.

9. The image display device according to claim 8,
wherein the background image generation section generates a vertical stripe pattern as the background image.

10. The image display device according to claim 1,
wherein in accordance with the behavior of the vehicle which is detected by the behavior detection section, the background image generation section generates the background image rotated to the left when the behavior indicates a left turn and also generates the background image rotated to the right when behavior indicates a right turn.

11. The image display device according to claim 1,
wherein the image transformation section trapezoidal-transforms the image in accordance with the behavior of the vehicle which is detected by the behavior detection section.

12. The image display device according to claim 11,
wherein in accordance with the behavior of the vehicle which is detected by the behavior detection section, the image transformation section trapezoidal-transforms the image by performing any of an enlargement and a reduction of at least one of a left end, a right end, a top end, and a bottom end of the image.

13. The image display device according to claim 1,
wherein the image transformation section enlarges or reduces the image.

14. The image display device according to claim 1,
wherein the composition section makes the composite image such that the background image generated by the background image generation section is placed in a background and the image transformed by the image transformation section is placed in a foreground.

15. The image display device according to claim 14,
wherein in accordance with the behavior of the vehicle which is detected by the behavior detection section, the composition section changes display positions of the background image generated by the background image generation section and of the image transformed by the image transformation section.

16. The image display device according to claim 1, further comprising a background image setting section for setting the background image generation section for generating the background image.

17. The image display device according to claim 16,
wherein the background image setting section selects a type of the background image.

18. The image display device according to claim 16 or claim 17,
wherein based on the behavior of the vehicle which is detected by the behavior detection section, the background image setting section sets a degree of changing a display position of the background image.

19. The image display device according to claim 18,

wherein based on the behavior of the vehicle which is detected by the behavior detection section, the background image setting section changes and sets, depending on a display position provided on the display section, the degree of changing the display position of the background image.

20. The image display device according to claim 1, further comprising an image transformation setting section for setting the image transformation section for transforming the image.

21. The image display device according to claim 20,
wherein the image transformation setting section sets the image transformation section to perform any one of a trapezoidal transformation, a reduction, and no transformation on the image to be transformed.

22. The image display device according to claim 21,
wherein, when the image transformation section is set to perform the trapezoidal transformation on the image to be transformed, the image transformation setting section sets a shape and a reduction ratio of the trapezoid.

23. The image display device according to claim 20,
wherein based on the behavior of the vehicle which is detected by the behavior detection section, the image transformation setting section sets a degree of transforming the image.

24. An image display device comprising:

a behavior detection section for detecting a behavior of a vehicle;
a background image generation section for generating a background image which moves based on the behavior detected by the behavior detection section;
an image transformation section for reducing an image;
a composition section for making a composite image of the background image generated by the background image generation section and the image reduced by the image transformation section; and
a display section for displaying the composite image made by the composition section.

25. An image display device comprising:

a behavior detection section for detecting a behavior of a vehicle;
an image transformation section for transforming an image based on the behavior detected by the behavior detection section; and
a display section for displaying the image transformed by the image transformation section.

26. A vehicle including the image display device according to any one of claim 1 through claim 25.

EP 1 977 931 A1

F I G. 2

201    202

IMAGE

F I G. 3

TRAVELING DIRECTION

ω

VELOCITY v

ANGULAR VELOCITY ω

RADIUS R

CENTRIFUGAL
ACCELERATION α

301

F I G. 4

u (RIGHTWARD MOVEMENT: POSITIVE)

402

401

403

ω (LEFTWARD ROTATION
: POSITIVE)

0

F I G. 5

u (RIGHTWARD MOVEMENT: POSITIVE)

502

501

503

ω (LEFTWARD ROTATION
: POSITIVE)

0

.

EP 1 977 931 A1

FIG. 6

u (RIGHTWARD MOVEMENT: POSITIVE)

R:
LARGE — 602

— 601

R: REFERENCE

— 603

R: SMALL

ω (LEFTWARD ROTATION
: POSITIVE)

0

FIG. 7

201   702

IMAGE

.

29

F I G. 8

```
            ┌──────────────────────────┐
            │          START           │
            └──────────────────────────┘
                         │
                         │◄─────────────────────┐
                         ▼                       │
  S801    ┌──────────────────────────────────┐  │
          │     DETECT BEHAVIOR OF VEHICLE    │  │
          └──────────────────────────────────┘  │
                         │                       │
                         ▼                       │
  S802    ┌──────────────────────────────────┐  │
          │ CHANGE DISPLAY POSITION OF        │  │
          │ BACKGROUND IMAGE IN ACCORDANCE    │  │
          │ WITH BEHAVIOR OF VEHICLE          │  │
          └──────────────────────────────────┘  │
                         │                       │
                         ▼                       │
  S803    ┌──────────────────────────────────┐  │
          │     TRANSFORM (REDUCE) IMAGE      │  │
          └──────────────────────────────────┘  │
                         │                       │
                         ▼                       │
  S804    ┌──────────────────────────────────┐  │
          │ MAKE COMPOSITE IMAGE OF           │  │
          │ BACKGROUND IMAGE AND IMAGE        │  │
          └──────────────────────────────────┘  │
                         │                       │
                         ▼                       │
  S805    ┌──────────────────────────────────┐  │
          │     DISPLAY COMPOSITE IMAGE       │  │
          └──────────────────────────────────┘  │
                         │                       │
                         ▼                       │
  S806         ◇ IS IMAGE DISPLAY ◇             │
             ◇ DEVICE IN OPERATION MODE ◇  Yes  │
                      ◇ ? ◇──────────────────────┘
                         │
                         │ No
                         ▼
            ┌──────────────────────────┐
            │           END            │
            └──────────────────────────┘
```

F I G. 9

(a)

YAW ANGULAR VELOCITY

901

TIME [sec]

(b)

YAW ANGULAR VELOCITY AT TYPICAL INTERSECTIONS

902

INTERSECTION 1
INTERSECTION 2
INTERSECTION 3
INTERSECTION 4
AVERAGE

TIME [sec]

F I G. 1 0

NUMBER OF PEOPLE

DATA INTERVAL 0 0.125 0.25 0.375 0.5 0.625 0.75 0.875

Ratio1

F I G. 1 1

F I G. 1 2

F I G. 1 3

F I G. 1 4

(a)

1402   1401

IMAGE

(b)

1404   1403

IMAGE

(c)

1406   1405

IMAGE

(d)

1408   1407

IMAGE

F I G.  1 5

k  (GREATER THAN 1 WHEN RIGHT END IS LARGER THAN LEFT END)

ω  (LEFTWARD ROTATION : POSITIVE)

F I G. 1 6

k (GREATER THAN 1 WHEN RIGHT END IS LARGER THAN LEFT END)

F I G. 1 7

k (GREATER THAN 1 WHEN RIGHT END IS LARGER THAN LEFT END)

R:
LARGE

1702

1701

R: REFERENCE

1703

R: SMALL

1

0

ω (LEFTWARD ROTATION
: POSITIVE)

F I G. 1 8

(a)

(b)

F I G.  1 9

F I G.  2 0

F I G. 2 1

F I G. 2 2

```
        ┌─────────────────────────┐
        │          START          │
        └─────────────────────────┘
                     │
                     ▼ ◄──────────────────────┐
S2201   ┌─────────────────────────┐           │
        │ DETECT BEHAVIOR OF VEHICLE │          │
        └─────────────────────────┘           │
                     │                         │
                     ▼                         │
S2202   ┌─────────────────────────┐           │
        │ GENERATE BACKGROUND IMAGE │          │
        └─────────────────────────┘           │
                     │                         │
                     ▼                         │
S2203   ┌─────────────────────────┐           │
        │ TRANSFORM IMAGE IN       │           │
        │ ACCORDANCE WITH BEHAVIOR │           │
        │ OF VEHICLE               │           │
        └─────────────────────────┘           │
                     │                         │
                     ▼                         │
S2204   ┌─────────────────────────┐           │
        │ MAKE COMPOSITE IMAGE OF   │          │
        │ BACKGROUND IMAGE AND IMAGE │         │
        └─────────────────────────┘           │
                     │                         │
                     ▼                         │
S2205   ┌─────────────────────────┐           │
        │ DISPLAY COMPOSITE IMAGE   │          │
        └─────────────────────────┘           │
                     │                         │
                     ▼                         │
S2206         ◇─────────────◇   Yes           │
        IS IMAGE DISPLAY  ───────────────────┘
        DEVICE IN OPERATION MODE
               ?
                     │ No
                     ▼
        ┌─────────────────────────┐
        │           END           │
        └─────────────────────────┘
```

F I G. 2 3

F I G. 2 4

FIG. 25

2501  2502

IMAGE

FIG. 26

2501  2602

IMAGE

## F I G. 2 7

```
            ┌─────────────────────────┐
            │          START          │
            └─────────────────────────┘
                         │
                         ▼ ◄─────────────────────────┐
  S2701 ──┐  ┌─────────────────────────┐             │
          └──│ DETECT BEHAVIOR OF VEHICLE│            │
             └─────────────────────────┘             │
                         │                            │
                         ▼                            │
  S2702 ──┐  ┌─────────────────────────────────┐     │
          └──│ CHANGE DISPLAY POSITION OF       │     │
             │ BACKGROUND IMAGE IN ACCORDANCE   │     │
             │ WITH BEHAVIOR OF VEHICLE         │     │
             └─────────────────────────────────┘     │
                         │                            │
                         ▼                            │
  S2703 ──┐  ┌─────────────────────────────────┐     │
          └──│ TRANSFORM IMAGE IN               │     │
             │ ACCORDANCE WITH BEHAVIOR         │     │
             │ OF VEHICLE                       │     │
             └─────────────────────────────────┘     │
                         │                            │
                         ▼                            │
  S2704 ──┐  ┌─────────────────────────────────┐     │
          └──│ MAKE COMPOSITE IMAGE OF          │     │
             │ BACKGROUND IMAGE AND IMAGE       │     │
             └─────────────────────────────────┘     │
                         │                            │
                         ▼                            │
  S2705 ──┐  ┌─────────────────────────────────┐     │
          └──│ DISPLAY COMPOSITE IMAGE          │     │
             └─────────────────────────────────┘     │
                         │                            │
                         ▼                            │
  S2706 ──┐       ╱─────────────────╲          Yes    │
          └──────╱   IS IMAGE DISPLAY  ╲──────────────┘
                 ╲ DEVICE IN OPERATION MODE ╱
                  ╲        ?        ╱
                         │
                         │ No
                         ▼
            ┌─────────────────────────┐
            │           END           │
            └─────────────────────────┘
```

F I G. 2 8

FIG. 29

TV VIEWING CONDITION

DISCOMFORT

3RD EMBODIMENT
CONDITION (ACTUAL
EXPERIMENT 2)

NORMAL CONDITION

TIME (min)

**INTERNATIONAL SEARCH REPORT**

| | International application No. |
|---|---|
| | PCT/JP2007/051093 |

A.   CLASSIFICATION OF SUBJECT MATTER
*B60R11/02*(2006.01)i, *B60R16/02*(2006.01)i, *G09G5/00*(2006.01)i, *G09G5/36*
(2006.01)i, *H04N5/66*(2006.01)i

According to International Patent Classification (IPC) or to both national classification and IPC

B.   FIELDS SEARCHED

Minimum documentation searched (classification system followed by classification symbols)
B60R11/02, B60R16/02, G09G5/00, G09G5/36, H04N5/66

Documentation searched other than minimum documentation to the extent that such documents are included in the fields searched
Jitsuyo Shinan Koho          1922-1996     Jitsuyo Shinan Toroku Koho    1996-2007
Kokai Jitsuyo Shinan Koho     1971-2007     Toroku Jitsuyo Shinan Koho    1994-2007

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| X<br>Y | JP 2-147446 A  (Hitachi, Ltd.),<br>06 June, 1990 (06.06.90),<br>Full text; all drawings<br>(Family: none) | 1-3,25,26<br>4-24 |
| Y<br>A | WO 2006/001295 A  (Matsushita Electric<br>Industrial Co., Ltd.),<br>05 January, 2006 (05.01.06),<br>Full text; all drawings<br>& JP 2006-007867 A | 4-24<br>1-3,25,26 |
| Y<br>A | JP 2005-294954 A  (Pioneer Electronic Corp.),<br>20 October, 2005 (20.10.05),<br>Full text; all drawings<br>(Family: none) | 7-24<br>1-6,25,26 |

| ☒   Further documents are listed in the continuation of Box C. | ☐   See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>10 April, 2007 (10.04.07) | Date of mailing of the international search report<br>24 April, 2007 (24.04.07) |
|---|---|
| Name and mailing address of the ISA/<br>   Japanese Patent Office | Authorized officer |
| Facsimile No. | Telephone No. |

Form PCT/ISA/210 (second sheet) (April 2005)

**INTERNATIONAL SEARCH REPORT**

| | International application No. |
|---|---|
| | PCT/JP2007/051093 |

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2003-154900 A  (Pioneer Electronic Corp.),<br>27 May, 2003 (27.05.03),<br>Full text; all drawings<br>& US 2003/0095179 A1 | 1-26 |
| P,A | WO 2006/011321 A1  (Matsushita Electric Industrial Co., Ltd.),<br>02 February, 2006 (02.02.06),<br>Full text; all drawings<br>& JP 2006-035980 A | 1-26 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002154350 A

- JP 2003154900 A

**Non-patent literature cited in the description**

- **TORU MATSUNAGA ; NORIAKI TAKEDA.** Motion Sickness and Space Sickness. *Practica Oto-Rhi-no-Laryngologica,* vol. 81 (8), 1095-1120